# EUROPEAN PATENT APPLICATION

(11) **EP 1 088 898 A1**
(43) Date of publication of application: **04.04.2001**
(21) Application number: 99118385.6
(22) Date of filing: 16.09.1999
(51) Int. Cl.: C12Q 1/44

(54) **A new assay to detect substances useful for the therapy of cancer and infectious diseases**

(71) Applicant: Max-Planck-Gesellschaft zur Förderung der Wissenschaften e.V., 80539 München (DE)
(72) Inventor: Nielsen, Erik, 69181 Leimen (DE); Christophoridis, Savvas, 69126 Heidelberg (DE); Murphy, Carol, 45500 Anatoli, Ioannina (GR); Zerial, Marino, 69124 Heidelberg (DE)
(74) Representative: Dehmel, Albrecht, Dr.

(57) **Abstract**

The present invention relates to the use of effectors/regulators for Rab and Rho GTPases in *in vitro* and *in vivo* assays that recapitulate and measure the role of these effectors/regulators in membrane transport and membrane-cytoskeleton interactions in the endocytic pathway as novel targets to find therapeutical drugs to prevent or inhibit cancer cell growth and arrest cancer cell invasiveness as well as for stimulating and/or restoring endocytic transport and phagosome maturation in cells infected by intracellular parasites, which drugs are therefore useful for the therapy and optionally also the prophylaxis of cancer and infectious diseases. In addition, the present invention is also directed to kits useful as a means to detect drugs suitable as anti-cancer and anti-infectious diseases drugs.

SEQUENCE LISTING

<110> Max-Planck-Gesellschaft

<120> A new assay to detect substances useful for the therapy
      of cancer and infectious diseases

<130> GI-003 EP

<140> xx
<141> 1999-09-16

<160> 12

<170> PatentIn Ver. 2.1

<210> 1
<211> 3510
<212> DNA
<213> Homo sapiens

<400> 1
atggcggaag aggaggtggc caagttggag aagcacttga tgcttctgcg gcaggagtat 60
gtcaagctgc agaagaagct ggcggagaca gagaagcgct gcgctctctt ggctgcgcag 120
gcaaacaagg agagcagcag cgagtccttc atcagccgtc tgctggccat cgtggcagac 180
ctctacgagc aggagcagta cagcgatctg aagataaagg ttggggacag gcacatcagt 240
gctcacaagt ttgtcctggc agcccgcagt gacagctgga gtctggctaa cttgtcttcc 300
actaaagagt tggacctgtc cgatgctaat cctgaggtga cgatgacaat gcttcgctgg 360
atctatacag atgagctgga gttcagagag gatgatgtgt tcctgactga actgatgaaa 420
ctagcaaatc ggtttcagct acagctcctc agggagagat gtgagaaggg tgttatgtct 480
ctagtgaatg tcaggaactg tattcgcttc taccagacgg cagaggagct gaatgccagc 540
acattgatga actactgtgc agaaattatt gcaagtcatt gggacgacct gaggaaggag 600
gatttcagca gcatgagcgc tcagttgtta tacaaaatga tcaaatccaa gacagagtac 660
ccgctacata aagccatcaa agtggagaga gaagacgtgg tcttcctgta tctgattgaa 720
atggattccc agctccctgg gaagctgaat gaagcggatc ataacggaga tctggcatta 780
gatctagccc tttcacgacg actggagagt attgccacca cgctggttag tcacaaagct 840
gatgtggaca tggtggacaa gagtggctgg agcttgttac acaaaggaat ccaaagagga 900
gatctctttg ctgccacttt cctcattaag aatggggcct ttgtcaacgc tgctacactg 960
ggtgcccagg agacaccact gcaccttgtg gccttgtaca gttcaaagaa acactcagca 1020
gatgtgatgt ctgagatggc gcagattgca gaggcccttc tgcaggctgg tgccaacccc 1080
aacatgcagg acagcaaggg gaggactcct ttacatgtgt ccatcatggc cgggaatgaa 1140
tatgtgttca gtcagctgct gcagtgcaaa caactagatt tagaactcaa agaccacgag 1200
ggcagcacgg ctctgtggct ggcagtgcag catatcacag tgtcttctga ccagtctgtg 1260
aaccccttcg aagatgtccc cgtggtaaat gggacttcat ttgatgagaa cagctttgca 1320
gccagactca tccagcgcgg cagccacaca gacgcacctg acacggcgac aggaaactgt 1380
ttactacagc gggcagctgg agcaggaaac gaggcagcag ctcttttcct ggcaaccaac 1440

ggtgcccatg tcaaccacag aaacaagtgg ggagaaaccc cgttgcacac agcgtgtcgg 1500
catggcctgg ccaacctcac agcagagctc ctgcagcaag gcgccaaccc aaacctgcag 1560
acggaggaag ctctgcctct gccaaaggag gccgcatccc tgaccagctt ggcggacagc 1620
gtccatctgc agacgccact gcacatggcg atcgcctata accatccgga tgtggtgtct 1680
gtcatcctgg agcagaaagc caatgctctt catgccacca acaacttgca gatcattccg 1740
gacttcagcc tcaaagattc ccgagaccag actgtgctgg gcctggcatt atggactggc 1800
atgcacacga tcgcagccca gctgctgggc tctggagctg ccatcaatga caccatgtcg 1860
gatgggcaga cgctactgca catggccata cagcggcagg acagcaagag cgcactcttc 1920
ctgctggagc accaggcaga tataaatgtc aggactcagg acggggagac agccctccag 1980
ctggccatca gaaaccagct tccactcgta gttgatgcca tatgcacccg aggagctgac 2040
atgtctgtgc cagatgagaa ggggaacccc ccgctgtggc ttgcattggc aaacaatctg 2100
gaggacatcg catccactct ggtcagacat ggctgtgatg ccacatgctg gggtccggga 2160
cctggtgggt gccttcagac gctcctgcac agagccattg atgaaaacaa cgagcccacc 2220
gcctgctttc ttattcgcag tggctgtgac gtgaacagtc ccagacaacc aggcgccaat 2280
ggagaaggag aggaagaggc tagagatggg cagacccctt tgcatttggc agcctcttgg 2340
gggctggaag agacagtaca gtgtcttctg gagtttggtg ccaacgtgaa cgcacaggat 2400
gcagaaggaa gaacccccat ccacgtggcc atcagcagcc aacacggtgt catcattcag 2460
ctgttggttt ctcaccccga tatccatttg aatgtacgag acagacaagg gctgaccccg 2520
tttgcctgtg ccatgacttt caagaacaac aagtcagccg aggccattct caaacgagag 2580
tccggggctg ctgagcaggt ggataacaag ggccggaatt tccttcatgt ggcagttcag 2640
aactctgata ttgaaagtgt gctgttcctg atcagtgtcc acgctaatgt gaattcaaga 2700
gtccaggatg cctccaagtt gacccccctg cacctcgctg tccaagcagg ctcagaaatt 2760
attgtccgca atttgcttct tgcgggagcc aaagtgaacg aattaaccaa gcatcgccag 2820
actgccctcc atcttgctgc ccagcaggac ctgcccacca tctgctcagt cctcctagag 2880
aatggcgtgg actttgctgc cgtggatgag aatggaaaca atgctcttca tcttgctgtc 2940
atgcacggcc ggctcaacaa catccgggtt ctcctgacag agtgcacagt ggacgccgaa 3000
gcctttaatc tcagaggcca gtcaccactg cacattttgg gacaatatgg caaggagaat 3060
gcagcggcca tctttgatct cttcctagaa tgcatgccgg ggtatcctct ggacaagccg 3120
gatgcagacg gcagcacggt gctgctcctg gcatacatga aggggaacgc caacttgtgc 3180
cgcgccatcg tccggtcggg ggctcgcctc ggggtgaata acaaccaggg agtcaacatc 3240
ttcaactacc aggtcgccac caagcagctc ctgttccgac tgctggatat gctgtccaag 3300
gagcctccgt ggtgtgacgg ctcctactgc tatgagtgca ctgccaggtt cggagtcacc 3360
actcgcaaac accactgtcg tcactgcgga cgtcttcttt gccataaatg ctcgaccaag 3420
gagattccta ttataaagtt tgatctgaac aagcctgtgc gggtttgcaa catttgtttt 3480
gatgtactga ctctgggtgg agtctcttga 3510

<210> 2
<211> 1169
<212> PRT
<213> Homo sapiens

<400> 2
Met Ala Glu Glu Glu Val Ala Lys Leu Glu Lys His Leu Met Leu Leu
1               5                   10                  15

Arg Gln Glu Tyr Val Lys Leu Gln Lys Lys Leu Ala Glu Thr Glu Lys
            20                  25                  30

Arg Cys Ala Leu Leu Ala Ala Gln Ala Asn Lys Glu Ser Ser Ser Glu
        35                  40                  45

Ser Phe Ile Ser Arg Leu Leu Ala Ile Val Ala Asp Leu Tyr Glu Gln
    50                  55                  60

Glu Gln Tyr Ser Asp Leu Lys Ile Lys Val Gly Asp Arg His Ile Ser
65                  70                  75                  80

Ala His Lys Phe Val Leu Ala Ala Arg Ser Asp Ser Trp Ser Leu Ala
                85                  90                  95

Asn Leu Ser Ser Thr Lys Glu Leu Asp Leu Ser Asp Ala Asn Pro Glu
            100                 105                 110

Val Thr Met Thr Met Leu Arg Trp Ile Tyr Thr Asp Glu Leu Glu Phe
        115                 120                 125

Arg Glu Asp Asp Val Phe Leu Thr Glu Leu Met Lys Leu Ala Asn Arg
    130                 135                 140

Phe Gln Leu Gln Leu Leu Arg Glu Arg Cys Glu Lys Gly Val Met Ser
145                 150                 155                 160

Leu Val Asn Val Arg Asn Cys Ile Arg Phe Tyr Gln Thr Ala Glu Glu
                165                 170                 175

Leu Asn Ala Ser Thr Leu Met Asn Tyr Cys Ala Glu Ile Ile Ala Ser
            180                 185                 190

His Trp Asp Asp Leu Arg Lys Glu Asp Phe Ser Ser Met Ser Ala Gln
        195                 200                 205

Leu Leu Tyr Lys Met Ile Lys Ser Lys Thr Glu Tyr Pro Leu His Lys
    210                 215                 220

Ala Ile Lys Val Glu Arg Glu Asp Val Val Phe Leu Tyr Leu Ile Glu
225                 230                 235                 240

Met Asp Ser Gln Leu Pro Gly Lys Leu Asn Glu Ala Asp His Asn Gly
                245                 250                 255

Asp Leu Ala Leu Asp Leu Ala Leu Ser Arg Arg Leu Glu Ser Ile Ala

            260                 265                 270

Thr Thr Leu Val Ser His Lys Ala Asp Val Asp Met Val Asp Lys Ser
        275                 280                 285

Gly Trp Ser Leu Leu His Lys Gly Ile Gln Arg Gly Asp Leu Phe Ala
    290                 295                 300

Ala Thr Phe Leu Ile Lys Asn Gly Ala Phe Val Asn Ala Ala Thr Leu
305                 310                 315                 320

Gly Ala Gln Glu Thr Pro Leu His Leu Val Ala Leu Tyr Ser Ser Lys
                325                 330                 335

Lys His Ser Ala Asp Val Met Ser Glu Met Ala Gln Ile Ala Glu Ala
            340                 345                 350

Leu Leu Gln Ala Gly Ala Asn Pro Asn Met Gln Asp Ser Lys Gly Arg
        355                 360                 365

Thr Pro Leu His Val Ser Ile Met Ala Gly Asn Glu Tyr Val Phe Ser
    370                 375                 380

Gln Leu Leu Gln Cys Lys Gln Leu Asp Leu Glu Leu Lys Asp His Glu
385                 390                 395                 400

Gly Ser Thr Ala Leu Trp Leu Ala Val Gln His Ile Thr Val Ser Ser
                405                 410                 415

Asp Gln Ser Val Asn Pro Phe Glu Asp Val Pro Val Val Asn Gly Thr
            420                 425                 430

Ser Phe Asp Glu Asn Ser Phe Ala Ala Arg Leu Ile Gln Arg Gly Ser
        435                 440                 445

His Thr Asp Ala Pro Asp Thr Ala Thr Gly Asn Cys Leu Leu Gln Arg
    450                 455                 460

Ala Ala Gly Ala Gly Asn Glu Ala Ala Ala Leu Phe Leu Ala Thr Asn
465                 470                 475                 480

Gly Ala His Val Asn His Arg Asn Lys Trp Gly Glu Thr Pro Leu His
                485                 490                 495

Thr Ala Cys Arg His Gly Leu Ala Asn Leu Thr Ala Glu Leu Leu Gln
            500                 505                 510

Gln Gly Ala Asn Pro Asn Leu Gln Thr Glu Glu Ala Leu Pro Leu Pro
        515                 520                 525

Lys Glu Ala Ala Ser Leu Thr Ser Leu Ala Asp Ser Val His Leu Gln
    530                 535                 540

Thr Pro Leu His Met Ala Ile Ala Tyr Asn His Pro Asp Val Val Ser
545                 550                 555                 560

Val Ile Leu Glu Gln Lys Ala Asn Ala Leu His Ala Thr Asn Asn Leu
                565                 570                 575

Gln Ile Ile Pro Asp Phe Ser Leu Lys Asp Ser Arg Asp Gln Thr Val
            580                 585                 590

Leu Gly Leu Ala Leu Trp Thr Gly Met His Thr Ile Ala Ala Gln Leu
        595                 600                 605

Leu Gly Ser Gly Ala Ala Ile Asn Asp Thr Met Ser Asp Gly Gln Thr
    610                 615                 620

Leu Leu His Met Ala Ile Gln Arg Gln Asp Ser Lys Ser Ala Leu Phe
625                 630                 635                 640

Leu Leu Glu His Gln Ala Asp Ile Asn Val Arg Thr Gln Asp Gly Glu
                645                 650                 655

Thr Ala Leu Gln Leu Ala Ile Arg Asn Gln Leu Pro Leu Val Val Asp
            660                 665                 670

Ala Ile Cys Thr Arg Gly Ala Asp Met Ser Val Pro Asp Glu Lys Gly
        675                 680                 685

Asn Pro Pro Leu Trp Leu Ala Leu Ala Asn Asn Leu Glu Asp Ile Ala
    690                 695                 700

Ser Thr Leu Val Arg His Gly Cys Asp Ala Thr Cys Trp Gly Pro Gly
705                 710                 715                 720

Pro Gly Gly Cys Leu Gln Thr Leu Leu His Arg Ala Ile Asp Glu Asn
                725                 730                 735

Asn Glu Pro Thr Ala Cys Phe Leu Ile Arg Ser Gly Cys Asp Val Asn
            740                 745                 750

Ser Pro Arg Gln Pro Gly Ala Asn Gly Glu Gly Glu Glu Glu Ala Arg
        755                 760                 765

Asp Gly Gln Thr Pro Leu His Leu Ala Ala Ser Trp Gly Leu Glu Glu
    770                 775                 780

Thr Val Gln Cys Leu Leu Glu Phe Gly Ala Asn Val Asn Ala Gln Asp
785                 790                 795                 800

Ala Glu Gly Arg Thr Pro Ile His Val Ala Ile Ser Ser Gln His Gly
                805                 810                 815

Val Ile Ile Gln Leu Leu Val Ser His Pro Asp Ile His Leu Asn Val
            820                 825                 830

Arg Asp Arg Gln Gly Leu Thr Pro Phe Ala Cys Ala Met Thr Phe Lys
        835                 840                 845

Asn Asn Lys Ser Ala Glu Ala Ile Leu Lys Arg Glu Ser Gly Ala Ala
    850                 855                 860

Glu Gln Val Asp Asn Lys Gly Arg Asn Phe Leu His Val Ala Val Gln
865                 870                 875                 880

Asn Ser Asp Ile Glu Ser Val Leu Phe Leu Ile Ser Val His Ala Asn
                885                 890                 895

Val Asn Ser Arg Val Gln Asp Ala Ser Lys Leu Thr Pro Leu His Leu
            900                 905                 910

Ala Val Gln Ala Gly Ser Glu Ile Ile Val Arg Asn Leu Leu Leu Ala
        915                 920                 925

Gly Ala Lys Val Asn Glu Leu Thr Lys His Arg Gln Thr Ala Leu His
    930                 935                 940

Leu Ala Ala Gln Gln Asp Leu Pro Thr Ile Cys Ser Val Leu Leu Glu
945                 950                 955                 960

Asn Gly Val Asp Phe Ala Ala Val Asp Glu Asn Gly Asn Asn Ala Leu
                965                 970                 975

His Leu Ala Val Met His Gly Arg Leu Asn Asn Ile Arg Val Leu Leu
            980                 985                 990

Thr Glu Cys Thr Val Asp Ala Glu Ala Phe Asn Leu Arg Gly Gln Ser

995                 1000                1005

Pro Leu His Ile Leu Gly Gln Tyr Gly Lys Glu Asn Ala Ala Ala Ile
    1010                1015                1020

Phe Asp Leu Phe Leu Glu Cys Met Pro Gly Tyr Pro Leu Asp Lys Pro
1025                1030                1035                1040

Asp Ala Asp Gly Ser Thr Val Leu Leu Leu Ala Tyr Met Lys Gly Asn
                1045                1050                1055

Ala Asn Leu Cys Arg Ala Ile Val Arg Ser Gly Ala Arg Leu Gly Val
            1060                1065                1070

Asn Asn Asn Gln Gly Val Asn Ile Phe Asn Tyr Gln Val Ala Thr Lys
        1075                1080                1085

Gln Leu Leu Phe Arg Leu Leu Asp Met Leu Ser Lys Glu Pro Pro Trp
    1090                1095                1100

Cys Asp Gly Ser Tyr Cys Tyr Glu Cys Thr Ala Arg Phe Gly Val Thr
1105                1110                1115                1120

Thr Arg Lys His His Cys Arg His Cys Gly Arg Leu Leu Cys His Lys
                1125                1130                1135

Cys Ser Thr Lys Glu Ile Pro Ile Ile Lys Phe Asp Leu Asn Lys Pro
            1140                1145                1150

Val Arg Val Cys Asn Ile Cys Phe Asp Val Leu Thr Leu Gly Gly Val
        1155                1160                1165

Ser

<210> 3
<211> 3390
<212> DNA
<213> Homo sapiens

<400> 3
atggcggaag aggaggtggc caagttggag aagcacttga tgcttctgcg gcaggagtat 60
gtcaagctgc agaagaagct ggcggagaca gagaagcgct gcgctctctt ggctgcgcag 120
gcaaacaagg agagcagcag cgagtccttc atcagccgtc tgctggccat cgtggcagac 180

ctctacgagc aggagcagta cagcgctaat cctgaggtga cgatgacaat gcttcgctgg 240
atctatacag atgagctgga gttcagagag gatgatgtgt tcctgactga actgatgaaa 300
ctagcaaatc ggtttcagct acagctcctc agggagagat gtgagaaggg tgttatgtct 360
ctagtgaatg tcaggaactg tattcgcttc taccagacgg cagaggagct gaatgccagc 420
acactgatga actactgtgc agaaattatt gcaagtcatt gggacgacct gaggaaggag 480
gatttcagca gcatgagcgc tcagttgtta tacaaaatga tcaaatccaa gacagagtac 540
ccgctacata aagccatcaa agtggagaga gaagacgtgg tcttcctgta tctgattgaa 600
atggattccc agctccctgg gaagctgaat gaagcggatc ataacggaga tctggcatta 660
gatctagccc tttcacgacg actggagagt attgccacca cgctggttag tcacaaagct 720
gatgtggaca tggtggacaa gagtggctgg agcttgttac acaaaggaat ccaaagagga 780
gatctctttg ctgccacttt cctcattaag aatggggcct ttgtcaacgc tgctacactg 840
ggtgcccagg agacaccact gcaccttgtg gccttgtaca gttcaaagaa acactcagca 900
gatgtgatgt ctgagatggc gcagattgca gaggcccttc tgcaggctgg tgccaacccc 960
aacatgcagg acagcaaggg gaggactcct ttacatgtgt ccatcatggc cgggaatgaa 1020
tatgtgttca gtcagctgct gcagtgcaaa caactagatt tagaactcaa agaccacgag 1080
ggcagcacgg ctctgtggct ggcagtgcag catatcacag tgtcttctga ccagtctgtg 1140
aaccccttcg aagatgtccc cgtggtaaat gggacttcat ttgatgagaa cagctttgca 1200
gccagactca tccagcgcgg cagccacaca gacgcacctg acacggcgac aggaaactgt 1260
ttactacagc gggcagctgg agcaggaaac gaggcagcag ctcttttcct ggcaaccaac 1320
ggtgcccatg tcaaccacag aaacaagtgg ggagaaaccc cgttgcacac agcgtgtcgg 1380
catggcctgg ccaacctcac agcagagctc ctgcagcaag gcgccaaccc aaacctgcag 1440
acggaggaag ctctgcctct gccaaaggag gccgcatccc tgaccagctt ggcggacagc 1500
gtccatctgc agacgccact gcacatggcg atcgcctata accatccgga tgtggtgtct 1560
gtcatcctgg agcagaaagc caatgctctt catgccacca acaacttgca gatcattccg 1620
gacttcagcc tcaaagattc ccgagaccag actgtgctgg gcctggcatt atggactggc 1680
atgcacacga tcgcagccca gctgctgggc tctggagctg ccatcaatga caccatgtcg 1740
gatgggcaga cgctactgca catggccata cagcggcagg acagcaagag cgcactcttc 1800
ctgctggagc accaggcaga tataaatgtc aggactcagg acggggagac agccctccag 1860
ctggccatca gaaaccagct tccactcgta gttgatgcca tatgcacccg aggagctgac 1920
atgtctgtgc cagatgagaa ggggaacccc ccgctgtggc ttgcattggc aaacaatctg 1980
gaggacatcg catccactct ggtcagacat ggctgtgatg ccacatgctg gggtccggga 2040
cctggtgggt gccttcagac gctcctgcac agagccattg atgaaaacaa cgagcccacc 2100
gcctgctttc ttattcgcag tggctgtgac gtgaacagtc ccagacaacc aggcgccaat 2160
ggagaaggag aggaagaggc tagagatggg cagacccctt tgcatttggc agcctcttgg 2220
gggctggaag agacagtaca gtgtcttctg gagtttggtg ccaacgtgaa cgcacaggat 2280
gcagaaggaa gaacccccat ccacgtggcc atcagcagcc aacacggtgt catcattcag 2340
ctgttggttt ctcaccccga tatccatttg aatgtacgag acagacaagg gctgaccccg 2400
tttgcctgtg ccatgacttt caagaacaac aagtcagccg aggccattct caaacgagag 2460
tccggggctg ctgagcaggt ggataacaag ggccggaatt tccttcatgt ggcagttcag 2520
aactctgata ttgaaagtgt gctgttcctg atcagtgtcc acgctaatgt gaattcaaga 2580
gtccaggatg cctccaagtt gacccccctg cacctcgctg tccaagcagg ctcagaaatt 2640
attgtccgca atttgcttct tgcgggagcc aaagtgaacg aattaaccaa gcatcgccag 2700
actgccctcc atcttgctgc ccagcaggac ctgcccacca tctgctcagt cctcctagag 2760
aatggcgtgg actttgctgc cgtggatgag aatggaaaca atgctcttca tcttgctgtc 2820
atgcacggcc ggctcaacaa catccgggtt ctcctgacag agtgcacagt ggacgccgaa 2880
gcctttaatc tcagaggcca gtcaccactg cacattttgg gacaatatgg caaggagaat 2940

gcagcggcca tctttgatct cttcctagaa tgcatgccgg ggtatcctct ggacaagccg 3000
gatgcagacg gcagcacggt gctgctcctg gcatacatga aagggaacgc caacttgtgc 3060
cgcgccatcg tccggtcggg ggctcgcctc ggggtgaata acaaccaggg agtcaacatc 3120
ttcaactacc aggtcgccac caagcagctc ctgttccgac tgctggatat gctgtccaag 3180
gagcctccgt ggtgtgacgg ctcctactgc tatgagtgca ctgccaggtt cggagtcacc 3240
actcgcaaac accactgtcg tcactgcgga cgtcttcttt gccataaatg ctcgaccaag 3300
gagattccta ttataaagtt tgatctgaac aagcctgtgc gggtttgcaa catttgtttt 3360
gatgtactga ctctgggtgg agtctcttga 3390

<210> 4
<211> 1129
<212> PRT
<213> Homo sapiens

<400> 4
Met Ala Glu Glu Glu Val Ala Lys Leu Glu Lys His Leu Met Leu Leu
1               5                   10                  15

Arg Gln Glu Tyr Val Lys Leu Gln Lys Lys Leu Ala Glu Thr Glu Lys
            20                  25                  30

Arg Cys Ala Leu Leu Ala Ala Gln Ala Asn Lys Glu Ser Ser Ser Glu
        35                  40                  45

Ser Phe Ile Ser Arg Leu Leu Ala Ile Val Ala Asp Leu Tyr Glu Gln
    50                  55                  60

Glu Gln Tyr Ser Ala Asn Pro Glu Val Thr Met Thr Met Leu Arg Trp
65                  70                  75                  80

Ile Tyr Thr Asp Glu Leu Glu Phe Arg Glu Asp Asp Val Phe Leu Thr
                85                  90                  95

Glu Leu Met Lys Leu Ala Asn Arg Phe Gln Leu Gln Leu Leu Arg Glu
            100                 105                 110

Arg Cys Glu Lys Gly Val Met Ser Leu Val Asn Val Arg Asn Cys Ile
        115                 120                 125

Arg Phe Tyr Gln Thr Ala Glu Glu Leu Asn Ala Ser Thr Leu Met Asn
    130                 135                 140

Tyr Cys Ala Glu Ile Ile Ala Ser His Trp Asp Asp Leu Arg Lys Glu
145                 150                 155                 160

Asp Phe Ser Ser Met Ser Ala Gln Leu Leu Tyr Lys Met Ile Lys Ser
                165                 170                 175

Lys Thr Glu Tyr Pro Leu His Lys Ala Ile Lys Val Glu Arg Glu Asp
            180                 185                 190

Val Val Phe Leu Tyr Leu Ile Glu Met Asp Ser Gln Leu Pro Gly Lys
        195                 200                 205

Leu Asn Glu Ala Asp His Asn Gly Asp Leu Ala Leu Asp Leu Ala Leu
    210                 215                 220

Ser Arg Arg Leu Glu Ser Ile Ala Thr Thr Leu Val Ser His Lys Ala
225                 230                 235                 240

Asp Val Asp Met Val Asp Lys Ser Gly Trp Ser Leu Leu His Lys Gly
                245                 250                 255

Ile Gln Arg Gly Asp Leu Phe Ala Ala Thr Phe Leu Ile Lys Asn Gly
            260                 265                 270

Ala Phe Val Asn Ala Ala Thr Leu Gly Ala Gln Glu Thr Pro Leu His
        275                 280                 285

Leu Val Ala Leu Tyr Ser Ser Lys Lys His Ser Ala Asp Val Met Ser
    290                 295                 300

Glu Met Ala Gln Ile Ala Glu Ala Leu Leu Gln Ala Gly Ala Asn Pro
305                 310                 315                 320

Asn Met Gln Asp Ser Lys Gly Arg Thr Pro Leu His Val Ser Ile Met
                325                 330                 335

Ala Gly Asn Glu Tyr Val Phe Ser Gln Leu Leu Gln Cys Lys Gln Leu
            340                 345                 350

Asp Leu Glu Leu Lys Asp His Glu Gly Ser Thr Ala Leu Trp Leu Ala
        355                 360                 365

Val Gln His Ile Thr Val Ser Ser Asp Gln Ser Val Asn Pro Phe Glu
    370                 375                 380

Asp Val Pro Val Val Asn Gly Thr Ser Phe Asp Glu Asn Ser Phe Ala
385                 390                 395                 400

Ala Arg Leu Ile Gln Arg Gly Ser His Thr Asp Ala Pro Asp Thr Ala

                405                 410                 415

Thr Gly Asn Cys Leu Leu Gln Arg Ala Ala Gly Ala Gly Asn Glu Ala
            420                 425                 430

Ala Ala Leu Phe Leu Ala Thr Asn Gly Ala His Val Asn His Arg Asn
        435                 440                 445

Lys Trp Gly Glu Thr Pro Leu His Thr Ala Cys Arg His Gly Leu Ala
    450                 455                 460

Asn Leu Thr Ala Glu Leu Leu Gln Gln Gly Ala Asn Pro Asn Leu Gln
465                 470                 475                 480

Thr Glu Glu Ala Leu Pro Leu Pro Lys Glu Ala Ala Ser Leu Thr Ser
                485                 490                 495

Leu Ala Asp Ser Val His Leu Gln Thr Pro Leu His Met Ala Ile Ala
            500                 505                 510

Tyr Asn His Pro Asp Val Val Ser Val Ile Leu Glu Gln Lys Ala Asn
        515                 520                 525

Ala Leu His Ala Thr Asn Asn Leu Gln Ile Ile Pro Asp Phe Ser Leu
    530                 535                 540

Lys Asp Ser Arg Asp Gln Thr Val Leu Gly Leu Ala Leu Trp Thr Gly
545                 550                 555                 560

Met His Thr Ile Ala Ala Gln Leu Leu Gly Ser Gly Ala Ala Ile Asn
                565                 570                 575

Asp Thr Met Ser Asp Gly Gln Thr Leu Leu His Met Ala Ile Gln Arg
            580                 585                 590

Gln Asp Ser Lys Ser Ala Leu Phe Leu Leu Glu His Gln Ala Asp Ile
        595                 600                 605

Asn Val Arg Thr Gln Asp Gly Glu Thr Ala Leu Gln Leu Ala Ile Arg
    610                 615                 620

Asn Gln Leu Pro Leu Val Val Asp Ala Ile Cys Thr Arg Gly Ala Asp
625                 630                 635                 640

Met Ser Val Pro Asp Glu Lys Gly Asn Pro Pro Leu Trp Leu Ala Leu
                645                 650                 655

Ala Asn Asn Leu Glu Asp Ile Ala Ser Thr Leu Val Arg His Gly Cys
            660                 665                 670

Asp Ala Thr Cys Trp Gly Pro Gly Pro Gly Gly Cys Leu Gln Thr Leu
        675                 680                 685

Leu His Arg Ala Ile Asp Glu Asn Asn Glu Pro Thr Ala Cys Phe Leu
    690                 695                 700

Ile Arg Ser Gly Cys Asp Val Asn Ser Pro Arg Gln Pro Gly Ala Asn
705                 710                 715                 720

Gly Glu Gly Glu Glu Glu Ala Arg Asp Gly Gln Thr Pro Leu His Leu
                725                 730                 735

Ala Ala Ser Trp Gly Leu Glu Glu Thr Val Gln Cys Leu Leu Glu Phe
            740                 745                 750

Gly Ala Asn Val Asn Ala Gln Asp Ala Glu Gly Arg Thr Pro Ile His
        755                 760                 765

Val Ala Ile Ser Ser Gln His Gly Val Ile Ile Gln Leu Leu Val Ser
    770                 775                 780

His Pro Asp Ile His Leu Asn Val Arg Asp Arg Gln Gly Leu Thr Pro
785                 790                 795                 800

Phe Ala Cys Ala Met Thr Phe Lys Asn Asn Lys Ser Ala Glu Ala Ile
                805                 810                 815

Leu Lys Arg Glu Ser Gly Ala Ala Glu Gln Val Asp Asn Lys Gly Arg
            820                 825                 830

Asn Phe Leu His Val Ala Val Gln Asn Ser Asp Ile Glu Ser Val Leu
        835                 840                 845

Phe Leu Ile Ser Val His Ala Asn Val Asn Ser Arg Val Gln Asp Ala
    850                 855                 860

Ser Lys Leu Thr Pro Leu His Leu Ala Val Gln Ala Gly Ser Glu Ile
865                 870                 875                 880

Ile Val Arg Asn Leu Leu Leu Ala Gly Ala Lys Val Asn Glu Leu Thr
                885                 890                 895

Lys His Arg Gln Thr Ala Leu His Leu Ala Ala Gln Gln Asp Leu Pro
            900                 905                 910

Thr Ile Cys Ser Val Leu Leu Glu Asn Gly Val Asp Phe Ala Ala Val
        915                 920                 925

Asp Glu Asn Gly Asn Asn Ala Leu His Leu Ala Val Met His Gly Arg
    930                 935                 940

Leu Asn Asn Ile Arg Val Leu Leu Thr Glu Cys Thr Val Asp Ala Glu
945                 950                 955                 960

Ala Phe Asn Leu Arg Gly Gln Ser Pro Leu His Ile Leu Gly Gln Tyr
                965                 970                 975

Gly Lys Glu Asn Ala Ala Ala Ile Phe Asp Leu Phe Leu Glu Cys Met
            980                 985                 990

Pro Gly Tyr Pro Leu Asp Lys Pro Asp Ala Asp Gly Ser Thr Val Leu
        995                 1000                1005

Leu Leu Ala Tyr Met Lys Gly Asn Ala Asn Leu Cys Arg Ala Ile Val
    1010                1015                1020

Arg Ser Gly Ala Arg Leu Gly Val Asn Asn Asn Gln Gly Val Asn Ile
1025                1030                1035                1040

Phe Asn Tyr Gln Val Ala Thr Lys Gln Leu Leu Phe Arg Leu Leu Asp
                1045                1050                1055

Met Leu Ser Lys Glu Pro Pro Trp Cys Asp Gly Ser Tyr Cys Tyr Glu
            1060                1065                1070

Cys Thr Ala Arg Phe Gly Val Thr Thr Arg Lys His His Cys Arg His
        1075                1080                1085

Cys Gly Arg Leu Leu Cys His Lys Cys Ser Thr Lys Glu Ile Pro Ile
    1090                1095                1100

Ile Lys Phe Asp Leu Asn Lys Pro Val Arg Val Cys Asn Ile Cys Phe
1105                1110                1115                1120

Asp Val Leu Thr Leu Gly Gly Val Ser
                1125

<210> 5
<211> 2406
<212> DNA
<213> Homo sapiens

<400> 5
atggaacaaa aactcatctc agaagaggat ctgaatttat caattgaatt catggcttct 60
ctggacgacc caggggaagt gagggagggc ttcctctgcc ctctgtgcct gaaggatctg 120
cagtctttct atcagcttca ctcacattac gaggaagaac actcagggga agaccgtgat 180
gtcaaagggc aaattaaaag tcttgtccag aaggctaaaa aagcaaagga caggttgttg 240
aaacgagaag gggatgatcg agcagagtca gggacccaag gatatgagtc tttcagctat 300
ggaggggttg atccttacat gtgggaaccc caggagcttg gtgctgtgag gagccatctt 360
tccgacctca aaaaacaccg agctgctaga attgaccact atgttgtgga agtcaataaa 420
ctaataatca ggttagagaa gctcactgca tttgacagaa caaatactga gtctgcaaag 480
attcgagcaa tagaaaagtc tgtggtgcct tgggtcaacg accaggatgt ccctttctgt 540
ccagactgtg ggaataagtt cagcatccgg aaccgccgcc accactgccg cctctgcggg 600
tctattatgt gcaagaagtg tatggagctc atcagccttc ccttggcaaa caagctcacc 660
agtgccagca aggagtccct gagcacccac accagcccca gccagtcacc caacagtgtc 720
catggctccc gccgaggcag catcagcagc atgagcagtg tcagctcggt cctggatgag 780
aaggacgatg accggatccg ctgctgtaca cactgcaagg acacgctgct caagagagag 840
cagcagattg atgagaagga gcacacacct gacatcgtga agctctacga gaaattacga 900
ctttgcatgg agaaagttga ccagaaagct ccagaataca tcaggatggc agcatcatta 960
aatgctgggg agacaaccta cagtctggaa catgccagtg accttcgagt ggaagtgcag 1020
aaagtgtatg agctgataga cgctttaagt aagaagatct taaccttggg cttgaaccag 1080
gaccctccac cacatccaag caatttgcgg ctgcagagaa tgatcagata ctcagctaca 1140
ctttttgtgc aggaaaagtt gcttggtttg atgtcactgc cgaccaaaga acagtttgag 1200
gaactgaaaa agaaaaggaa ggaggaaatg gagaggaaga gggccgtgga gagacaagct 1260
gctctggagt cccagcgaag gcttgaggaa aggcagagtg gcctggcttc tcgagcggcc 1320
aacggggagg tggcatctct ccgcaggggc cctgccccct tgagaaaggc tgagggctgg 1380
ctcccactgt caggaggtca ggggcagagt gaggactcag acccgctcct ccagcagatc 1440
cacaacatca catcattcat caggcaggcc aaggccgcgg gccgcatgga tgaagtgcgc 1500
actctgcagg agaacctgcg gcagctgcag gacgagtatg accagcagca gacagagaag 1560
gccatcgagc tgtcccggag gcaggctgag gaggaggacc tgcagcggga acagctgcag 1620
atgttgcgtg aacgggagtt ggaacgagaa agggagcagt ttcgggtggc atccctgcac 1680
acacggactc ggtccctgga cttcagagaa atcggccctt ttcagctgga gcccagcaga 1740
gagcctcgca cccaccttgc ttatgctttg gatctaggct cttccccagt tccaagcagc 1800
acagctccca agaccccttc acttagctca actcaaccca ccagagtgtg gtctgggccc 1860
ccagccgttg gccaggagcg cttaccccag agcagcatgc cacagcaaca tgagggcccc 1920
tccttaaacc cctttgatga ggaagacctc tccagcccca tggaagaggc cactactggt 1980
cctcctgctg caggggtttc cttagaccct tcagcccgca tcctgaagga gtacaatcct 2040
ttcgaggaag aggacgagga ggaggaagca gtggcaggga atccattcat tcagccagac 2100
agcccagctc ctaacccctt cagtgaggaa gacgaacatc cccagcagag gctctcaagc 2160
cctctggttc ctggtaaccc ctttgaggaa cccacctgta tcaaccccct tgagatggac 2220
agtgacagtg ggccagaggc tgaggagccc atagaggaag agctcctcct gcagcagatc 2280
gataacatca aggcatacat ctttgatgcc aagcagtgcg gccgcctgga tgaggtagag 2340

gtgctgacag agaatctgcg ggagctgaag cacaccctgg ccaagcagaa ggggggcact 2400
gactga 2406

<210> 6
<211> 784
<212> PRT
<213> Homo sapiens

<400> 6
Met Ala Ser Leu Asp Asp Pro Gly Glu Val Arg Glu Gly Phe Leu Cys
1               5                   10                  15

Pro Leu Cys Leu Lys Asp Leu Gln Ser Phe Tyr Gln Leu His Ser His
            20                  25                  30

Tyr Glu Glu Glu His Ser Gly Glu Asp Arg Asp Val Lys Gly Gln Ile
        35                  40                  45

Lys Ser Leu Val Gln Lys Ala Lys Lys Ala Lys Asp Arg Leu Leu Lys
    50                  55                  60

Arg Glu Gly Asp Asp Arg Ala Glu Ser Gly Thr Gln Gly Tyr Glu Ser
65                  70                  75                  80

Phe Ser Tyr Gly Gly Val Asp Pro Tyr Met Trp Glu Pro Gln Glu Leu
                85                  90                  95

Gly Ala Val Arg Ser His Leu Ser Asp Phe Lys Lys His Arg Ala Ala
            100                 105                 110

Arg Ile Asp His Tyr Val Val Glu Val Asn Lys Leu Ile Ile Arg Leu
        115                 120                 125

Glu Lys Leu Thr Ala Phe Asp Arg Thr Asn Thr Glu Ser Ala Lys Ile
    130                 135                 140

Arg Ala Ile Glu Lys Ser Val Val Pro Trp Val Asn Asp Gln Asp Val
145                 150                 155                 160

Pro Phe Cys Pro Asp Cys Gly Asn Lys Phe Ser Ile Arg Asn Arg Arg
                165                 170                 175

His His Cys Arg Leu Cys Gly Ser Ile Met Cys Lys Lys Cys Met Glu
            180                 185                 190

Leu Ile Ser Leu Pro Leu Ala Asn Lys Leu Thr Ser Ala Ser Lys Glu
        195                 200                 205

Ser Leu Ser Thr His Thr Ser Pro Ser Gln Ser Pro Asn Ser Val His
    210                 215                 220

Gly Ser Arg Arg Gly Ser Ile Ser Ser Met Ser Ser Val Ser Ser Val
225                 230                 235                 240

Leu Asp Glu Lys Asp Asp Asp Arg Ile Arg Cys Cys Thr His Cys Lys
                245                 250                 255

Asp Thr Leu Leu Lys Arg Glu Gln Gln Ile Asp Glu Lys Glu His Thr
            260                 265                 270

Pro Asp Ile Val Lys Leu Tyr Glu Lys Leu Arg Leu Cys Met Glu Lys
        275                 280                 285

Val Asp Gln Lys Ala Pro Glu Tyr Ile Arg Met Ala Ala Ser Leu Asn
    290                 295                 300

Ala Gly Glu Thr Thr Tyr Ser Leu Glu His Ala Ser Asp Leu Arg Val
305                 310                 315                 320

Glu Val Gln Lys Val Tyr Glu Leu Ile Asp Ala Leu Ser Lys Lys Ile
                325                 330                 335

Leu Thr Leu Gly Leu Asn Gln Asp Pro Pro Pro His Pro Ser Asn Leu
            340                 345                 350

Arg Leu Gln Arg Met Ile Arg Tyr Ser Ala Thr Leu Phe Val Gln Glu
        355                 360                 365

Lys Leu Leu Gly Leu Met Ser Leu Pro Thr Lys Glu Gln Phe Glu Glu
    370                 375                 380

Leu Lys Lys Lys Arg Lys Glu Glu Met Glu Arg Lys Arg Xaa Val Glu
385                 390                 395                 400

Arg Gln Ala Ala Leu Glu Ser Gln Arg Arg Leu Glu Glu Arg Gln Ser
                405                 410                 415

Gly Leu Ala Ser Arg Ala Ala Asn Gly Glu Val Ala Ser Leu Arg Arg
            420                 425                 430

Gly Pro Ala Pro Leu Arg Lys Ala Glu Gly Trp Leu Pro Leu Ser Gly

435                 440                 445

Gly Gln Gly Gln Ser Glu Asp Ser Asp Pro Leu Leu Gln Gln Ile His
    450                 455                 460

Asn Ile Thr Ser Phe Ile Arg Gln Ala Lys Ala Ala Gly Arg Met Asp
465                 470                 475                 480

Glu Val Arg Thr Leu Gln Glu Xaa Leu Arg Gln Leu Gln Asp Glu Tyr
                485                 490                 495

Asp Gln Gln Gln Thr Glu Lys Ala Ile Glu Leu Ser Arg Arg Gln Ala
            500                 505                 510

Glu Glu Glu Asp Leu Gln Arg Glu Gln Leu Gln Met Leu Arg Glu Arg
        515                 520                 525

Glu Leu Glu Arg Glu Arg Glu Gln Phe Arg Val Ala Ser Leu His Thr
    530                 535                 540

Arg Thr Arg Ser Leu Asp Phe Arg Glu Ile Gly Pro Phe Gln Leu Glu
545                 550                 555                 560

Pro Ser Arg Glu Pro Arg Thr His Leu Ala Tyr Ala Leu Asp Leu Gly
                565                 570                 575

Ser Ser Pro Val Pro Ser Ser Thr Ala Pro Lys Thr Pro Ser Leu Ser
            580                 585                 590

Ser Thr Gln Pro Thr Arg Val Trp Ser Gly Pro Pro Ala Val Gly Gln
        595                 600                 605

Glu Arg Leu Pro Gln Ser Ser Met Pro Gln Gln His Glu Gly Pro Ser
    610                 615                 620

Leu Asn Pro Phe Asp Glu Glu Asp Leu Ser Ser Pro Met Glu Glu Ala
625                 630                 635                 640

Thr Thr Gly Pro Pro Ala Ala Gly Val Ser Leu Asp Pro Ser Ala Arg
                645                 650                 655

Ile Leu Lys Glu Tyr Asn Pro Phe Glu Glu Glu Asp Glu Glu Glu Glu
            660                 665                 670

Ala Val Ala Gly Asn Pro Phe Ile Gln Pro Asp Ser Pro Ala Pro Asn
        675                 680                 685

Pro Phe Ser Glu Glu Asp Glu His Pro Gln Gln Arg Leu Ser Ser Pro
    690                 695                 700

Leu Val Pro Gly Asn Pro Phe Glu Glu Pro Thr Cys Ile Asn Pro Phe
705                 710                 715                 720

Glu Met Asp Ser Asp Ser Gly Pro Glu Ala Glu Glu Pro Ile Glu Glu
                725                 730                 735

Glu Leu Leu Leu Gln Gln Ile Asp Asn Ile Lys Ala Tyr Ile Phe Asp
            740                 745                 750

Ala Lys Gln Cys Gly Arg Leu Asp Glu Val Glu Val Leu Thr Glu Asn
        755                 760                 765

Leu Arg Glu Leu Lys His Thr Leu Ala Lys Gln Lys Gly Gly Thr Asp
    770                 775                 780

<210> 7
<211> 1659
<212> DNA
<213> Homo sapiens

<400> 7
atgaagaaaa acagagaaag attctgcaat agagagagag aatttgtata taaatttaaa 60
gtaggaagtc agtgcttaga actgagagtg ccactcaaat ttcctgttca agagaatgcc 120
agtcatttgc atggacgtct gatgctgctg cacagtttac cgtgctttat agaaaaagac 180
ttaaaagaag ctctgactca gtttatagaa gaagaatccc tcagcgatta tgatagagat 240
gctgaagcat ccctggcagc tgtgaaatca ggtgaagtag atttacatca gctggcgagt 300
acatgggcca aagcttatgc tgagaccacg ttagagcatg caaggcctga agaacccagc 360
tgggatgaag attttgcaga tgtgtaccat gacttaattc attctcctgc ctctgaaact 420
ctcttaaatt tggaacataa ttactttgtt agtatctcag aactgattgg tgaaagagat 480
gtggagctga aaaaattacg agagagacaa ggtattgaaa tggaaaaagt catgcaggaa 540
ttgggaaaat cactgacaga tcaagatgta aattcactgg ctgctcagca ttttgaatcc 600
cagcaagacc tagaaaataa atggtcgaat gaattaaaac aatcaactgc catccaaaaa 660
caagagtatc aagaatgggt aataaaactt caccaagacc taaaaaaccc caacaacagc 720
tcccttagtg aggaaattaa agttcagcca agtcagttca gagaatctgt agaagcaatt 780
ggaaggattt atgaggaaca gagaaagtta gaagaaagtt ttaccattca cttaggagcc 840
cagttgaaga ccatgcataa tttgagattg ctgagagcag atatgctgga cttctgtaag 900
cataaaagaa atcatcgaag tggtgtgaaa cttcatcggc tccaaacagc tctgtcactt 960

tattctacat ctctctgtgg cctggtttta ctagtagata atcgaattaa ttcatatagt 1020
ggtattaaaa gagattttgc cacagtttgc caagaatgca ctgacttcca tttcccccga 1080
attgaagagc aattagaagt tgtccaacag gtggtacttt atgctagaac ccagcgcagg 1140
agtaaattga aagaatcact tgattctgga aaccaaaatg gaggaaatga tgataagact 1200
aagaatgctg agaggaacta tttaaatgtt ttacctgggg aattttatat tacacggcat 1260
tctaatctct cagaaatcca tgttgctttc catctctgtg tggatgacca tgtgaaatcg 1320
ggaaacatca ctgctcgtga tcctgccatt atgggactcc gaaatatact caaagtttgc 1380
tgtacccatg acatcacaac aataagcatt cctctcttgc tggtacatga tatgtcagag 1440
gaaatgacta taccctggtg cttaaggaga gcggaacttg tgttcaagtg tgtcaaaggt 1500
ttcatgatgg aaatggcttc atgggatgga ggaatttcta ggacagtgca atttctagta 1560
ccacagagta tttctgaaga aatgttttat caacttagta acatgcttcc ccagatcttc 1620
cgagtatcat caacactcac tctgacatcc aagcactaa 1659

<210> 8
<211> 552
<212> PRT
<213> Homo sapiens

<400> 8

<210> 9
<211> 1050
<212> DNA
<213> Homo sapiens

<400> 9
atgaaaggct tatatttcca acagagttcc acagatgaag aaataacatt tgtatttcaa 60
gaaaaggaag atcttcctgt tacagaggat aactttgtga aacttcaagt taaagcttgt 120
gctctgagcc agataaatac aaagcttctg gcagaaatga agatgaaaaa ggatttattt 180
cctgttggga gagaaattgc tggaattgta ttagatgttg gaagcaaggt atcattcttt 240
caaccagatg atgaagtagt tggaattttg cccctggact ctgaagaccc tggactttgt 300

gaagttgtta gagtacatga gcattacttg gttcataaac cagaaaaggt cacatggacg 360
gaagcagcag gaagcattcg ggatggagtg cgtgcctata cagctctgca ttatctttct 420
catctctctc ctggaaaatc agtgctgata atggatggag caagtgcatt tggtacaata 480
gctattcagt tagcacatca tagaggagcc aaagtgattt caacagcatg cagccttgaa 540
gataagcagt gccttgaaag attcagacct cccatagccc gagtgattga tgtatctaat 600
gggaaagttc atgttgctga aagctgtttg gaagaaacag gtggcctggg agtagatatt 660
gtcctagatg ctggagtgag attatatagt aaagatgatg aaccagctgt aaaactacaa 720
ctactaccac ataaacatga tatcatcaca cttcttggtg ttggaggcca ctgggtaaca 780
acagaagaaa accttcagtt ggatcctcca gatagccact gccttttcct caagggagca 840
acgttagctt tcctgaatga tgaagtttgg aatttgtcaa atgtacaaca gggaaaatat 900
ctttgtatct taaaggatgt gatggagaag ttatcaactg gtgttttcag acctcagttg 960
gatgaaccca ttccactgta tgaggcaaaa gtttccatgg aagctgttca gaaaaatcaa 1020
ggaagaaaaa agcaagttgt tcaattttaa 1050

<210> 10
<211> 349
<212> PRT
<213> Homo sapiens

<400> 10
Met Lys Gly Leu Tyr Phe Gln Gln Ser Ser Thr Asp Glu Glu Ile Thr
1               5                   10                  15

Phe Val Phe Gln Glu Lys Glu Asp Leu Pro Val Thr Glu Asp Asn Phe
            20                  25                  30

Val Lys Leu Gln Val Lys Ala Cys Ala Leu Ser Gln Ile Asn Thr Lys
        35                  40                  45

Leu Leu Ala Glu Met Lys Met Lys Lys Asp Leu Phe Pro Val Gly Arg
    50                  55                  60

Glu Ile Ala Gly Ile Val Leu Asp Val Gly Ser Lys Val Ser Phe Phe
65                  70                  75                  80

Gln Pro Asp Asp Glu Val Val Gly Ile Leu Pro Leu Asp Ser Glu Asp
                85                  90                  95

Pro Gly Leu Cys Glu Val Val Arg Val His Glu His Tyr Leu Val His
            100                 105                 110

Lys Pro Glu Lys Val Thr Trp Thr Glu Ala Ala Gly Ser Ile Arg Asp
        115                 120                 125

Gly Val Arg Ala Tyr Thr Ala Leu His Tyr Leu Ser His Leu Ser Pro

130                 135                 140

Gly Lys Ser Val Leu Ile Met Asp Gly Ala Ser Ala Phe Gly Thr Ile
145                 150                 155                 160

Ala Ile Gln Leu Ala His His Arg Gly Ala Lys Val Ile Ser Thr Ala
                165                 170                 175

Cys Ser Leu Glu Asp Lys Gln Cys Leu Glu Arg Phe Arg Pro Pro Ile
            180                 185                 190

Ala Arg Val Ile Asp Val Ser Asn Gly Lys Val His Val Ala Glu Ser
        195                 200                 205

Cys Leu Glu Glu Thr Gly Gly Leu Gly Val Asp Ile Val Leu Asp Ala
    210                 215                 220

Gly Val Arg Leu Tyr Ser Lys Asp Asp Glu Pro Ala Val Lys Leu Gln
225                 230                 235                 240

Leu Leu Pro His Lys His Asp Ile Ile Thr Leu Leu Gly Val Gly Gly
                245                 250                 255

His Trp Val Thr Thr Glu Glu Asn Leu Gln Leu Asp Pro Pro Asp Ser
            260                 265                 270

His Cys Leu Phe Leu Lys Gly Ala Thr Leu Ala Phe Leu Asn Asp Glu
        275                 280                 285

Val Trp Asn Leu Ser Asn Val Gln Gln Gly Lys Tyr Leu Cys Ile Leu
    290                 295                 300

Lys Asp Val Met Glu Lys Leu Ser Thr Gly Val Phe Arg Pro Gln Leu
305                 310                 315                 320

Asp Glu Pro Ile Pro Leu Tyr Glu Ala Lys Val Ser Met Glu Ala Val
                325                 330                 335

Gln Lys Asn Gln Gly Arg Lys Lys Gln Val Val Gln Phe
            340                 345

<210> 11
<211> 663
<212> DNA

<213> Homo sapiens

<400> 11
atgnnnnnng agcggctccc taacaggccc gcctgtctgc tcgtggccag cggcgccgcc 60
gaaggtgtgt cggcccagtc cttcctccac tgtttcacga tggccagcac cgccttcaac 120
ctgcaggtgg ccacccctgg ggggaaagcc atggaatttg tggatgtgac tgagagcaat 180
gcacgctggg tgcaagactt ccgcctcaag gcttacgcca gccccgccaa gctcgagtcc 240
atcgatggtg cccggtacca tgccctcctg atccccagct gtcctggggc cctgaccgac 300
ctggccagca gtggctccct ggcccgtatc ctgcagcact tccactctga gagcaaaccc 360
atctgcgccg tcggccacgg tgtcgctgcc ctgtgctgtg ccaccaacga ggacagatcc 420
tgggtgttcg acagctacag cctgacaggg ccctctgtgt gtgagctcgt caggcccccc 480
ggcttcgccc gcctgccgct cgtggtggag gacttcgtga aggattcggg cgcctgcttc 540
agtgggcttg gggcagcacc aggctggggc agaggaggaa agcaagagga cagacctcca 600
gaagagcagc ggagggcggg tgaggatttc cccatatacc agtgtgtgtt catctaccca 660
tga 663

<210> 12
<211> 220
<212> PRT
<213> Homo sapiens

<400> 12
Met Ala Ser Glu Arg Leu Pro Asn Arg Pro Ala Cys Leu Leu Val Ala
1               5                   10                  15

Ser Gly Ala Ala Glu Gly Val Ser Ala Gln Ser Phe Leu His Cys Phe
            20                  25                  30

Thr Met Ala Ser Thr Ala Phe Asn Leu Gln Val Ala Thr Pro Gly Gly
        35                  40                  45

Lys Ala Met Glu Phe Val Asp Val Thr Glu Ser Asn Ala Arg Trp Val
    50                  55                  60

Gln Asp Phe Arg Leu Lys Ala Tyr Ala Ser Pro Ala Lys Leu Glu Ser
65                  70                  75                  80

Ile Asp Gly Ala Arg Tyr His Ala Leu Leu Ile Pro Ser Cys Pro Gly
                85                  90                  95

Ala Leu Thr Asp Leu Ala Ser Ser Gly Ser Leu Ala Arg Ile Leu Gln
            100                 105                 110

His Phe His Ser Glu Ser Lys Pro Ile Cys Ala Val Gly His Gly Val
        115                 120                 125

Ala Ala Leu Cys Cys Ala Thr Asn Glu Asp Arg Ser Trp Val Phe Asp
    130                 135                 140

Ser Tyr Ser Leu Thr Gly Pro Ser Val Cys Glu Leu Val Arg Ala Pro
145                 150                 155                 160

Gly Phe Ala Arg Leu Pro Leu Val Val Glu Asp Phe Val Lys Asp Ser
                165                 170                 175

Gly Ala Cys Phe Ser Gly Leu Gly Ala Ala Pro Gly Trp Gly Arg Gly
            180                 185                 190

Gly Lys Gln Glu Asp Arg Pro Pro Glu Glu Gln Arg Arg Ala Gly Glu
        195                 200                 205

Asp Phe Pro Ile Tyr Gln Cys Val Phe Ile Tyr Pro
    210                 215                 220

## Description

The present invention relates to the use of effectors/regulators for Rab and Rho GTPases in *in vitro* and *in vivo* assays that recapitulate and measure the role of these effectors/regulators in membrane transport and membrane-cytoskeleton interactions in the endocytic pathway as novel targets to find therapeutical drugs to prevent or inhibit cancer cell growth and arrest cancer cell invasiveness as well as for stimulating and/or restoring endocytic transport and phagosome maturation in cells infected by intracellular parasites, which drugs are therefore useful for the therapy and optionally also the prophylaxis of cancer and infectious diseases. In addition, the present invention is also directed to kits useful as a means to detect drugs suitable as anti-cancer and anti-infectious diseases drugs.

The process of membrane transport can be operationally divided into five steps:
(1) the inclusion of cargo into a membrane patch,
(2) the formation and release of the vesicle from the donor compartment,
(3) the movement of the vesicle to the target compartment,
(4) the docking of the vesicle, and
(5) the fusion of the vesicle with the membrane of the target compartment allowing the release of the cargo.

Two classes of molecules have been shown to play an important role in the regulation of intracellular transport. The **first class** is represented by Rab proteins, small GTPases of the Ras superfamily, which are required in virtually every transport step which has been investigated (Novick and Zerial, 1997). These molecules use the conformational change induced by GTP hydrolysis to regulate the kinetics of membrane docking and fusion. They act therefore as molecular switches that, by recruiting and activating effector proteins, govern downstream events necessary for vesicle formation (step 2), docking and fusion (steps 4 and 5) (Novick and Zerial, 1997). Rab proteins also regulate the activity of SNAREs. SNAREs are integral membrane proteins that by pairing on opposite membranes engaged in docking lead to membrane fusion (Rothman, 1994). The pairing of SNAREs requires the activity of Rab proteins and therefore of Rab effectors. For example, endosome membrane docking requires the presence of the Rab5 effector EEA1 which acts upstream SNAREs (Christoforidis et al. 1999a).

Intracellular transport is also regulated by membrane-cytoskeleton interactions. The cytoskeleton is made by different types of filaments, of which actin filaments and microtubules are the best characterised. Actin filaments and microtubules undergo continuous remodeling to serve the needs of the cell in cell architecture, motility, and organelle movement. Microtubules play a pivotal role in cell division but also constitute tracks along which cargo is transported across long distances, for example between the periphery and the center of the cell, from the basal to the apical pole in epithelial cells, or along the axon in neurons. Actin filaments are well known to function in muscle contraction. They are also important for cell motility, cell shape and vesicular transport. Different proteins are necessary to attach the organelles and transport vesicles to, and move them along, these filaments. Recent data provides evidence that Rab proteins also regulate the association with and motility of vesicles along cytoskeletal filaments (step 3) (Novick and Zerial, 1997). However, **the second class** of molecules that plays a role in intracellular transport is represented by another branch of the superfamily of Ras-like small GTPases, Rho proteins. Rho family members are established regulators of the actin cytoskeleton (Hall, 1992) These proteins control various forms of actin assembly and modulate cellular responses in response to signal transduction, including sustaining tumoral transformation induced by oncogenes (Machesky and Hall, 1996). Rho proteins are also capable of regulating vesicle formation (step 2) and vesicle movement (step 3) by a mechanism that is not yet understood. In particular, a member of the Rho family GTPases, RhoD, has been identified and characterized, and it has been demonstrated that this GTPase regulates both the actin cytoskeleton and the intracellular motility of early endosomes (Murphy et al., 1996).

The present inventors' understanding of the mechanisms underlying membrane trafficking and membrane-cytoskeleton interactions, on the other hand, has also applications in biotechnological research. There are two main research areas for which this knowledge has proven to have important implications: cancer and infectious diseases.

Cell growth is regulated by growth factors that by binding specific receptors on the cell surface trigger a signaling cascade that culminates with the regulation of gene expression (Cantley et al., 1991; Sorkin and Waters, 1993). Growth factors and their receptors are transported in the endocytic pathway and several lines of evidence suggest that endocytic transport regulate the signalling response. First, growth factor receptors and their bound ligand are internalized into endosomes, and endocytic trafficking of these molecules plays a critical role not only in attenuating the signaling response but also in establishing and controlling specific signaling pathways (Vieira et al., 1996). Second, the importance of transport of signalling molecules along the endocytic pathway is underscored by the finding that some components of the signaling machinery are localized to endocytic organelles (Tsukazaki et al., 1998). Third, it is known since long time that in cancer cells, the oncogenic alterations in cell growth also result in alterations of membrane trafficking and membrane-cytoskeleton interactions (Bar-Sagi D, 1986; Veithen A, 1998). Indeed, many tumor cell properties correlate with alterations in cell organization. For example, metastasis is a multistep process that requires many cellular functions, including changes in cell adhesion and cell locomotion. Cell locomotion occurs by a complex mechanism involving the coordinated activity of both the cytoskeleton - actin and microtubules - and membrane trafficking - especially endocytic and recycling structures (Bretscher and Aguado-Velasco, 1998; Hopkins et al., 1994). Through their function in regulating the actin cytoskeleton, Rho proteins participate in the process of cell locomotion and in invasiveness (Habets GG, 1994; Michiels F, 1995). It is also important to consider that cancer cells require higher supply of nutrients and, therefore, tumor growth is accompanied by a deregulation of angiogenesis (Malonne et al., 1999). Clearly, the spreading of endothelial cells during angiogenesis that occurs concomitantly with tumor growth and the spreading of transformed cells requires an increase in the cell motility machinery. Altogether, these observations strongly suggest that the trafficking of signaling molecules through the endocytic pathway as well as the structural/functional properties of endosomes play a role in the signal transduction mechanism. Consequently, the function of molecules that play an important role in endocytic transport can be rate-limiting important factors for the survival and spreading of cancer cells.

Another area according to the present invention where membrane trafficking molecules, i.e., GTPase effector/regulator molecules, can serve as therapeutical targets is that of infectious diseases. It is well established that certain microbes can sabotage the cellular defense mechanisms and survive intracellularly. This is the case for example for *Mycobacterium tuberculosis* that once internalised by phagocytosis resides within the phagosome without coming in contact with harmful organelles such as the lysosomes (Deretic V, 1999). A possible strategy to facilitate the killing of the pathogen is to re-activate the membrane trafficking route, thus resuming the maturation of the phagosome and, therefore, the exposure of the internalised bacterium to low pH and harmful hydrolytic enzymes.

Based on these above experimental results, the object of the present invention was to develop an assay useful for the detection and identification of molecules (pharmaceutical drugs). In order to achieve this object, the present inventors developed the strategy that molecules (proteins) interacting with GTPases such as GTPases of the Rab and Rho family and, thus, functioning at the level of endosome trafficking and coordinating the interaction of this organelle with the cytoskeleton, can provide novel targets for molecules (pharmaceutical drugs) aimed at inhibiting cell locomotion and invasiveness (and thereby cancer) as well as infectious diseases. A further object of the present invention was to provide a kit comprising molecules interacting with GTPases (effector or regulator proteins/molecules; for exact definitions see further below), which kit is useful as a means for the detection and identification of pharmaceutical drugs exhibiting properties making them preferred drugs for combatting cancer and infectious diseases.

The following definitions are given in order to more fully specify the terms as used in the present application. **"Invasiveness"** refers to the spreading of cancer cells or other cells such as endothelial cells (rather than the invasion of bacteria). **"GTPase-interacting protein"** or **"regulator/regulatory protein/regulatory molecule"** refers to any protein that interacts with the GTPase under any nucleotide conformation and for any purpose. GTPase-interacting proteins/regulators regulate the nucleotide-bound state of the GTPase, i.e., the GTPase activating protein (GAP) interacts with the GTP-bound GTPase and downregulates the activity of the GTPase by stimulating the hydrolysis of GTP. A GDP/GTP exchange factor promotes nucleotide exchange, thereby activating the GTPase. **"Effector"** or **"effector/effector protein/effector molecule"** is defined to mean a protein that interacts with the GTPase only if the GTPase is in the GTP-bound, also called active, form, can stabilize this conformation and mediates the "effect" of that GTPase. Thus, both effectors and regulators may therefore also be termed GTPase effectors and GTPase regulators, respectively. Additionally, it is apparent for the skilled reader that the term **"regulator"** is broader than the term **"effector"** and includes the latter.

The above mentioned strategy implies the first step of employing known effectors/regulators or identifying novel effectors/regulators of Rab and Rho GTPases, or of other (small) GTPases, specifically regulating endosome trafficking and endosome-cytoskeleton interactions, and the additional step of designing an *in vitro* and/or *in vivo* assay to screen for inhibitors or activators of these effector/regulator molecules. In particular, the strategy includes the identification of substances useful as pharmaceutical drugs due to their interference with the process of cell locomotion, i.e., it includes the identification of inhibitors of membrane-cytoskeleton interactions.

The above object in mind, the present inventors established an assay system based on the use of such effectors/regulators, as defined hereinbefore, as targets for pharmaceutical drugs useful in the therapy of cancer and infectious diseases and any other disease which affects or requires the function of the endocytic/recycling trafficking machinery. According to a preferred embodiment of this aspect of the present invention, an assay is provided using both an effector/regulator and a Rab or Rho protein (or any other small GTPase). Accordingly, one aspect of the present invention is the use of the above mentioned GTPase effector/regulator molecules or proteins as targets in an *in vitro* or *in vivo* assay system to detect substances useful as a pharmaceutical/therapeutical agent for the prophylaxis and the treatment of cancer and infectious diseases. Another object of the present invention is a kit useful for carrying out the assay of the invention. The kit necessarily contains at least one type of effector/regulator molecule. The at least one GTPase effector/regulator molecule is either native and biochemically purified from a vertebrate, or recombinant and biochemically purified from bacterial cultures, from yeast cultures, or from other cultured eukaryotic cells, in either case labelled by a covalent modification or radioactivity suitable for use in the assay.

According to a preferred embodiment of this aspect of the present invention the kit additionally contains at least one type of GTPase and/or endosomal membrane fractions obtained by subcellular fractionation and labelled by internalization of transferrin, either fluorescently labeled or labeled by any other modification that allows its detection, and/or cytosolic extracts, and/or an ATP-regenerating system and/or a number of chemicals to be tested for their suitability as an anti-cancer or anti-infectious diseases drug. Other specific and preferred embodiments of both the kit and the use according to the present invention may be readily taken from the claims attached hereto.

Drugs with anti-cancer and/or anti-infectious diseases activity include those molecules (including peptides) mimicking the peptide structure of the respective target molecule. Typical representatives of the above classes are molecules/substances that carry one or more of the following functional groups: a halide atom bound to an alkyl, alkenyl, alkinyl or aryl residue, an alcohol group, an ether group, a carbonyl function (aldehyde or ketone), a carboxylic acid group, a carboxylic anhydride group, a carbamoyl group, a haloformyl group, a cyano group, an ester group including a lactone group, a benzyl, phenyl, tolyl, tosyl, sulfonyl group, an amino group (primary, secondary, tertiary), an isocyanate, a cyanate, a thioisocyanate, a thiocyanate, a carbamate, an azide, a diazo group, and a quinone group. Other representatives of suitable drugs are organometallic compounds, sterol moiety(ies)-containing molecules, and inorganic acids and complexes such as metallocenes, a cytokine, a hormone, or an oligopeptide comprising up to 20, preferably 8, 10, or 12, amino acid residues.

The advantage (and the gist) of the instant invention consists in (1) the development of a comprehensive screening method based on molecular networks, (2) the sensitivity of the assays, and (3) the selectivity of the targets. The activity of the factors under control of the central switch can be responsible for coordinating various interconnected functions such as (a) endocytosis, (b) the distribution, i.e., sorting of proteins and lipids within endosomes and from endosomes to other intracellular locations (e.g. interactions of phagosomes with endosomes and lysosomes), (c) the motility of endosomes along the cytoskeleton, and (d) cell migration. This multiplicity of factors responsible for a wide number of cellular functions is defined as *molecular network.* The present invention resides in enabling the skilled person to study the molecular network of many different GTPases, in particular five GTPases localized to endosomes, i.e., Rab5, Rab4, Rab11, Rab17, and RhoD (see below), as a whole in order to establish various (GTPase) effector/regulator proteins that are suitable as targets for pharmaceutical drugs. In this approach, the GTPase itself will not serve as a target molecule because its activity is so crucial for cell homeostasis that it would invariably result in toxic effects. Rather, the effector/regulator molecules specifically functioning in protein and lipid sorting, endosome motility and endosome-cytoskeleton interactions will be the target. For example, compounds that affect cell motility could be screened more specifically without affecting other important cellular functions (i.e. endocytosis). In the alternative, and according to a preferred embodiment of the present invention, the target may well be one of the effectors/regulators as defined above, but additionally a GTPase such as Rab5, Rab4, Rab11, Rab17, or RhoD will be present in the assay system.

The present inventors have functionally characterised several Rab GTPases (e.g., Rab5, Rab4, Rab11, and Rab17) and effectors/regulators of these GTPases, localised to the early endosomes and the recycling endosomes, which GTPases and effectors/regulators, respectively, are thus suitable for the assay according to the invention. These Rab proteins sequentially control transport in the endocytic and recycling pathway. Rab5 controls transport from the plasma membrane to early endosomes whereas Rab4 and Rab11 function in protein sorting and recycling from early and recycling endosomes back to the plasma membrane. The expression of Rab17 is restricted to polarized cells such as epithelial cells and neurons. This latter GTPase regulates trafficking through the apical recycling endosome, thus contributing to the generation and maintenance of cell polarity. In addition, the above mentioned RhoD that had been demonstrated to regulate the actin cytoskeleton and the intracellular motility of early endosomes has been characterised. Furthermore, an activated mutant form of RhoD, RhoD V26G, carrying a glycine rather than a valine residue at position 26 has been found by the present inventors to arrest cell motility. Thus, effectors/regulators of all these GTPases and the respective GTPases (in combination with their effectors/regulators) may be employed in accordance with the present invention in an *in vitro* or *in vivo* assay system as a target in order to screen for substances that are useful as a pharmaceutical agent for the prophylaxis and treatment of cancer (benign tumors, malignant tumors, carcinomas, sarcomas, melanomas, leukemias, gliomas, or neuroblastomas) and infectious diseases such as tuberculosis, cholera, malaria, pseudotuberculosis, gastroenteritis, enteric fever, typhus, and any other infectious diseases caused by an infectious agent that infects cells by the endocytic route and resides intracellularly in phagosomes escaping the cellular killing mechanisms (in this regard, it should be recalled that the GTPases per se and alone can not serve as a target for the drugs to be detected; rather, the target for the drug is an effector/regulator molecule). Other examples of infectious diseases are those diseases caused by the following pathogens (bacteria or organisms): Mycobacterium, Staphylococcus, Toxoplasma, Trypanosoma, Listeria, Salmonella, Legionella, Leishmania, Coxiella, Shigella, Yersinia, Neisseria, Vibrio, Bartonella, and any other intracellular pathogen that resides in intracellular phagosomes and escapes the cellular killing mechanisms.

As the proteins described herein, i.e., the Rab, Rho proteins and their effectors/regulators, are, unless exceptions, ubiquitously expressed, pharmaceutical agents for the prophylaxis and treatment of tumors of any cellular origin can be screened for and detected according to the present invention. Specifically, the following tumors may be mentioned: lung carcinoma, osteosarcoma, lymphoma, leukemia, soft tissue sarcoma, breast carcinoma, bile cancer, cervix carcinoma, cancer of the (small) intestine, of the kidneys, of the cavity of the mouth, penis carcinoma, ovary cancer, stomach cancer, cancer of the tongue, brain cancer, bladder carcinoma, prostate carcinoma, liver carcinoma, and carcinoma of the pancreas and every tumor that invades other tissues and organs.

Drugs with anti-cancer and/or anti-infectious diseases activity include those molecules (including peptides) mimicking the peptide structure of the respective target molecule. Typical representatives of the above classes are molecules/substances that carry one or more of the following functional groups: a halide atom bound to an alkyl, alkenyl, alkinyl or aryl residue, an alcohol group, an ether group, a carbonyl function (aldehyde or ketone), a carboxylic acid group, a carboxylic anhydride group, a carbamoyl group, a haloformyl group, a cyano group, an ester group including a lactone group, a benzyl, phenyl, tolyl, tosyl, sulfonyl group, an amino group (primary, secondary, tertiary), an isocyanate, a cyanate, a thioisocyanate, a thiocyanate, a carbamate, an azide, a diazo group, and a quinone group. Other representatives of suitable drugs are organometallic compounds, sterol moiety(ies)-containing molecules, and inorganic acids and complexes such as metallocenes, a cytokine, a hormone, or an oligopeptide comprising up to 20, preferably 8, 10, or 12, amino acid residues.

To elucidate the molecular mechanism whereby these GTPases exert their function in membrane trafficking and cytoskeleton organization, the present inventors have developed a method to biochemically identify Rab5 protein effector molecules (Chistoforidis et al., 1999a). This method is based on the previously described affinity chromatography (Amano et al., 1996) but has been optimized to result in the specific elution and large-scale purification of Rab effectors and GTPase interacting proteins in general, in amounts sufficient for both their identification by microsequencing techniques and their functional characterization (Chistoforidis et al., 1999a).

In addition to that method, the present inventors have developed a novel method. This method is described in Example 3 below. With this method, the inventors have re-purified several of the proteins that bind Rab5. Using this modified affinity chromatography procedure, more than 20 effector and/or regulator proteins interacting directly or indirectly with Rab5 (Figure 1a in Christoforidis et al., 1999a) have been identified and demonstrated to exhibit functional activity as shown by their ability to substitute cytosol in an *in vitro* endosome fusion reaction (Chistoforidis et al., 1999a). Some of these molecules such as Rabex-5 regulate the activity of Rab5 (i.e., its nucleotide state) (Horiuchi et al., 1997), others such as Rabaptin-5 and EEA1 participate in the endosome docking and fusion process (Stenmark et al., 1995). Still others regulate the ability of endosomes to move along microtubules, and all of these proteins are suitable candidates as a target for the detection of chemical drugs (compounds) that may turn out to be highly efficient anti-cancer and/or anti-infectious disease agents.

In addition, expression of an activated mutant of Rab5 (Rab5Q79L) induces an increased recruitment of actin on the expanded endosomes suggesting that Rab5 either directly or indirecly regulates the attachment/nucleation of actin filaments with the endosome membrane. Based on the above data, in particular in view of this large number of Rab5 interacting molecules (more than 20), the present inventors concluded that the activity of Rab5 is not restricted to endocytic membrane docking and fusion. Most importantly, they also concluded that modulating the activity of Rab5, and therefore of its effectors/regulators, would result in changes in organelle motility *in vivo* and, consequently, in cell motility. This is perfectly consistent with the effect observed by the present inventors that expression of activated Rab5 leads to an increase in motility of fibroblasts and endothelial cells, implicating this GTPase in the regulation of cell migration. The effector/regulator protein(s) responsible for this activity (to regulate membrane trafficking and membrane-cytoskeleton interactions including the ability of endosomes to move along microtubules) are therefore, in accordance with the present invention, specific targets for drugs aimed at inhibiting the ability of the Rab5 machinery to sustain cell migration.

Similar experiments have been and are being conducted on two other GTPases functioning in endosome trafficking, Rab4 and Rab11, as well as on the small GTPase RhoD. Several candidate proteins for Rab4 effectors/regulators have been purified and one of these proteins has been sequenced and identified as the human homolog of the yeast Adenylyl Cyclase-associated protein CAP (Field et al., 1990). This protein is a component of the Ras-activated cyclase complex in yeast and therefore required for the proliferative signal in yeast. In addition, this protein has been implicated in the interaction with the actin cytoskeleton, since the carboxy terminus of CAP binds actin (Zelicof et al., 1996).

Given the role of Rab and Rho proteins in the regulation of membrane transport and membrane-cytoskeleton interactions, the effectors/regulators for these small GTPases are targets for pharmaceutical drugs aimed at (1) inhibiting cell invasiveness and (2) enhancing cell-defense mechanisms against intracellular pathogens, such drugs therefore being valuable weapons in the battle against tumor (and cancer) and infectious diseases. For example, the opposite behaviour of Rab5 and RhoD on cell motility opens interesting possibilities for identifying drugs that targeted against the specific effectors/regulators of these GTPases: one can either inhibit the stimulatory activity of Rab5 or enhance the inhibitory activity of RhoD on cell motility, in order to obtain the same effect - reduced cell motility.

A strategy similar to that established and described above for Rab5 has been applied for other Rab and Rho proteins regulating endocytic trafficking. For example, the small GTPases Rab4, Rab11, and Rab17 reside onto and regulate trafficking through the early and recycling endosomes. Given that in motile cells recycling endosomes preferentially deliver membranes to the leading edge of the cell (Bretscher, 1992; Bretscher and Aguado-Velasco, 1998; Hopkins et al., 1994) and given the established functional role of Rab proteins, it is very likely that Rab4 and Rab11 characterized by the present inventors may participate in the regulation of cell motility given their localization to endosomes. Therefore, the effectors/regulators of these GTPases will also represent novel potential targets according to the instant invention.

Effectors/regulators of still another GTPase, RhoD, which GTPase is localized to the plasma membrane and early endosomes (Murphy et al., 1996), are being purified and characterised by the present inventors by means of the above described method(s). Additionally, this GTPase (RhoD) has been found by the present inventors to counteract the stimulatory effect of Rab5 on cell motility. The Rho family of GTPases, in particular RhoD, is involved in the regulation of the actin cytoskeleton organization and, hence, in processes relevant to cell locomotion. Accordingly, when overexpressed these GTPases (particularly RhoD) induce actin polymerization and inhibit early endosome dynamics by affecting the intracellular motility of this organelle. Furthermore, cell motility studies have shown that expression of the above mentioned activated RhoD mutant (RhoD V26G) also blocks the motility of fibroblasts and endothelial cells. RhoD effectors/regulators are thus molecules that control the motility of endosomes, presumably via the interaction with actin filaments, and whose activities are necessary in the process of cell motility, and highly suitable targets for the detection of drugs effective in the treatment of cancer and infectious diseases, in particular in the treatment of the cancers and diseases listed above.

By applying affinity chromatography similar to that described in the literature for Rab5 (Christoforidis et al., 1999a) on RhoD, the present inventors have been successful in purifying candidate effectors/regulators of RhoD. The molecules have been subjected to mass spectrometry sequence analysis to elucidate their identity.

On the other hand, further Rab and Rho effectors/regulators to be identified and characterised in the future will also act in various aspects of endosome function, including endosome and cell motility, and endosome-phagosome interactions. An approach to identify further effectors/ regulators has been described by Zerial and colleagues (Chistoforidis et al., 1999a), and the procedure described by the authors is similarly suitable to detect, identify, and characterise further, as yet unknown, effector/regulator molecules (proteins) of small GTPases of the Rab and Rho families.

The multiplicity of Rab5 (or any other of the GTPases) effector/regulator molecules serves to execute distinct functions in endosome dynamics and cell dynamics all coordinated by the GTPase switch of Rab5 (or any other of the GTPases). It is therefore a "package" also referred above as network of molecules that is necessary to coordinate different functions. These molecules can operationally be divided into 6 classes on the basis of either their established identity and/or function and on the basis of their possible function for which different claims in drug discovery can be proposed. The effectors/regulators of all classes may be used for the assay as defined herein, i.e., for the detection of suitable drugs for the treatment of cancer and infectious diseases.

### Class 1

### Representative effectors/regulators: Rabaptin-5, Rabaptin-5b, Rabex-5, EEA1, and PI 3-K

### Function: endocytic trafficking

### Assay: endosome fusion, endosome motility

Factors that play a role in (i) the regulation of the nucleotide cycle of Rab5: Rabex-5 (Horiuchi et al., 1997), in (ii) endosome docking and fusion: Rabaptin-5 (Stenmark et al., 1995), Rabaptin-5b (Gournier et al., 1998), and EEA1 (Chistoforidis et al., 1999a; Simonsen et al., 1998), or in (iii) the regulation of this process via the generation of phosphoinositide-3-phosphate (PI 3-K) which is necessary for the membrane recruitment of EEA1 (Christoforidis et al., 1999b), have been identified previously. Among a large number of effector/regulator proteins for Rab5 two distinct phosphatidyl-inositol 3-kinases (PI 3-K), hVPS34 and p85a/p110b, have been determined. The two kinases are differentially distributed along the endocytic pathway, and the activity of hVPS34 is specifically required for EEA1 membrane recruitment and endosome fusion. It has also been demonstrated that VPS34 regulates the motility of endosomes along microtubules, suggesting that PI 3-K activity is necessary to coordinate the function of various Rab5 effectors/regulators, including those controlling membrane-cytoskeleton interactions. PI 3-K are therefore considered to represent a particularly suitable target of drugs that block the motility activity. Similarly, targets other than PI 3-K may be identified among the Rab5, Rab4, and Rab11 effectors/regulators that couple the activity in membrane docking and fusion with the organelle dynamics. One such target coupling the activity in membrane docking and fusion with the organelle dynamics is Rabaptin-5 due to its capacity to couple Rab4 to Rab5 function.

The class 1 effector/regulator molecules may be used according to the present invention as drug targets in cancer on the basis of regulating cell motility and invasiveness. The inventors of the present invention have found out that Rab5 regulates the motility of endothelial cells in vitro (this result was the outcome of experiments described in the Examples below). Since the Rabaptin-5/Rabex-5 complexes regulate the nucleotide cycle of Rab5, and since the kinases PI 3-K regulate the recruitment of effectors/regulators, these molecules have been identified as targets for drugs aimed at inhibiting the activity of Rab5 in cell motility. The class 1 effector/regulator molecules may also be used as drug targets in cancer on the basis of the signalling molecules regulating cell growth. Growth factors and growth factor receptors are transported through the endosomes before being degraded in the late endosomes and lysosomes. Molecules that regulate the trafficking of receptors along the endocytic pathway can be used as targets for drugs aimed at interfering with transport (i.e., clearance from the plasma membrane, inhibition of recycling, transport to endosomes where signalling molecules are located (Tsukazaki et al., 1998), increase transport to degradative compartments where receptor-ligand complexes are degraded) and, consequently, inhibiting mitogenic signalling. Finally, the class 1 effector/regulator molecules may also be used as drug targets in infectious diseases (see the above list exemplifying infectious diseases particularly suitable according to the present invention). The small GTPase Rab5 has been observed to accumulate on *Mycobacterium tuberculosis* phagosomes (Deretic V, 1997). Interestingly, the Rab5 effector Rabaptin-5 is not present on the phagosome membrane. Thus, it must be concluded that there are differences between endosomes and phagosomes in the utilization of the Rab5 effectors/regulators. In the case of *Listeria monocytogenes* phagosomes, it has been observed that an upregulation in expression of the small GTPase Rab5 occurs in the course of infection of macrophages by a *Listeria monocytogenes* non-lytic mutant (Alvarez-Dominguez et al., 1996; Alvarez-Dominguez and Stahl, 1999; Alvarez-Dominguez and Stahl, 1998). This increased Rab5 recruitment results in an increased fusogenicity of the phagosomes with endosomes leading to intracellular killing of the bacterium. Accordingly, in order to detect and identify substances suitable for the theurapeutic treatment of pathogen infections, the Rab5 effector/regulator responsible for this effect may be used as a target in the assay described in detail further below. In this case, the upregulation of the host cell defense mechanism could be accomplished by a suitable drug. The activator of Rab5, Rabex-5, that functions as a nucleotide exchange factor and converts the protein into the GTP-bound active form (Horiuchi et al., 1997) is a particularly suitable drug target in this regard. By enhancing its activity (by means of a drug activating Rabex-5), the inventors have been able to up-regulate the Rab5 machinery and facilitate phagosome-endosome interactions. Alternatively, other Rab5 effectors/regulators (still to be identified) playing a role in endosome trafficking could serve as targets.

### Class 2

### Representative effectors/regulators: Rabankyrin-5 and p110 FYVE

### Function: endocytic trafficking, possible interaction with the actin cytoskeleton .

### Assay: endosome fusion, endosome motility

This class includes at least two proteins which share the same PI (3)P-binding domain, the FYVE-finger. One protein is the human homolog of mouse Ankhzn (Ito et al., 1999), to be renamed Rabankyrin-5, given its ability to bind Rab5. The present inventors also found a Rabankyrin-5 splice variant (SV). Rabankyrin-5 contains ankyrin repeats. It is localized to early endosomes, binds Rab5, and is suspected to play a role in interaction with the actin cytoskeleton. As Rab5 regulates also the association with, or nucleation of, actin filaments on the endosome membrane, Rabankyrin-5 is one of the effectors useful as a target according to the invention as it regulates the interaction with actin filaments and, therefore, plays a role in endosome motility.

The sequence of the gene coding for human Rabankyrin-5 is depicted in the sequence listing attached to this application (SEQ ID NO: 1), whereas the sequence of the protein itself is given in the sequence listing as SEQ ID NO: 2. The nucleotide and amino acid sequences of the SV gene and protein are indicated in the sequence listing as SEQ ID NO: 3 and SEQ ID NO: 4, respectively.

The second molecule is temporarily named p110FYVE. It is localized to early endosomes as well, binds Rab5 as well, and is also known to be complexed with the mammalian homolog of yeast Vps45p, a protein involved in transport to the vacuole (Peterson et al., 1999). p110FYVE probably plays a role in the regulation of transport and participates in the movement of endosomes along the actin and microtube cytoskeleton. The nucleotide and amino acid sequences of p110FYVE are given in SEQ ID NO: 5 and SEQ ID NO: 6, respectively.

### Class 3

### Representative effector/regulator: hHook (Kramer and Phistry, 1996)

### Function: endocytic trafficking, in particular transport to degradative compartments

### Assay: endosome fusion, endosome motility

hHook is the mammalian homolog of the Drosophila gene hook, which is involved in endocytosis and receptor signalling. This molecule interacts either directly with Rab5 or indirectly through another Rab5-binding protein. Given the function of Hook in receptor trafficking and that this process is important for receptor signalling during the developmental process in Drosophila, the human Hook is most likely an important molecule controlling receptor signalling also in mammalian cells. Its function is expected to be important in cancer and therefore constitutes a novel drug target by virtue of its classification as another Rab5 effector/regulator.

### Class 4

### Representative effectors/regulators: multi-protein complex including p100, p95, p60, p45, p20

### Function: endocytic trafficking, possibly in polarized cells such as epithelial cells and neurons

### Assay: endosome fusion, endosome motility

A complex of 5 proteins (p100, p95, p60, p45, p25) has been isolated. At least one of these molecules, or the entire complex, interacts either directly with Rab5 or indirectly through another Rab5-binding protein. Their function is in polarized endocytic trafficking, by analogy with the exocyst, a multi-protein complex involved in polarized secretion to the yeast bud (TerBush et al., 1996).

The nucleotide and amino acid sequences of three proteins of the complex have been detemined (p60: SEQ ID NO: 7 and SEQ ID NO: 8; p45: SEQ ID NO: 9 and SEQ ID NO: 10; p25: SEQ ID NO: 11 and SEQ ID NO: 12, respectively).

### Class 5

### Representative effectors/regulators: unknown

### Function: endocytic trafficking, intracellular distribution of endosomes, motility of endosomes along microtubules, cellular motility

### Assay: attachment of endosomes on microtubules, endosome motility along microtubules

This class represents a microtubule motor or a molecule that in turn regulates a microtubule motor. The present inventors have found that Rab5 regulates the motility of early endosomes *in vivo* and both the attachment of early endosomes to microtubules and the motility of early endosomes along microtubules *in vitro.* In order to study Rab5-positive early endosome dynamics *in vivo,* wild type Rab5 fused to the C-terminus of enhanced-green-fluorescent-protein (EGFP) was expressed in stably transformed A431 cells. EGFP-Rab5 was observed primarily on punctate cytoplasmic structures distributed throughout the cells as well as accumulated in the juxtanuclear region. These structures colocalized with fluorescently labeled transferrin and with the early-endosome associated protein, EEA1, and were therefore established to be endosomes. In order to visualize the dynamics of the EGFP-Rab5 compartment and test for the role of microtubules in this process, we utilized video-enhanced fluorescence microscopy. Observed were two distinct types of movement of EGFP-Rab5 endosomes in the cell. First, a short-range, non-linear motion was most evident in the cell periphery, or in lamellipodia. The second type of motion observed was found primarily in the cell body, and consisted of long-range, linear movements. These long-range motions were often bi-directional and were abolished when microtubules were depolymerized with nocodazole. In cells expressing high levels of EGFP-Rab5, early endosomes accumulated in a juxtanuclear region. Using video microscopy it was observed that this redistribution correlated with increased endosome movement towards the center of the cell. This observation is consistent with the inventors' previous studies showing that overexpression of wild type or constitutively active Rab5 results in accumulation of enlarged endosomes in the perinuclear region (Bucci et al., 1992; Stenmark et al., 1994). For experimental details see Example 4.

**Rab5 stimulates association of early endosomes with microtubules *in vitro.*** The effect of Rab5 on endosome motility implies that this GTPase may regulate the association of endosomes with microtubules. The present inventors developed an assay that measures the attachment of early endosomes to microtubules *in vitro.* Purified early endosomes were mixed with cytosol and taxol-stabilized microtubules. Microtubules were then sedimented through a sucrose cushion to separate endosomes associated with microtubules from unattached endosomes and cytosolic components, and the pellet fraction was subjected to immunoblot analysis. Cytoskeletal proteins present in the cytosol did not contribute significantly to the sedimentation of endosomes as almost no transferrin receptor was detected in the pellet without addition of microtubules. Recruitment of endosomes to microtubules was stimulated by cytosol. Although recruitment was further enhanced in the presence of millimolar ATP concentrations nucleotide was not essential for association, as depletion of ATP with hexokinase and glucose did not reduce endosome recruitment to levels lower than cytosol alone. We next tested whether absence or presence of Rab5 effected the recruitment of endosomes on microtubules. Addition of RabGDI alone at a concentration (1µM) that removes Rab5 from endosomal membranes, resulted in a significant reduction of endosomes in the microtubule pellet. To test the effect of higher levels of Rab5 on endosomes we added Rab5-RabGDI complex at concentrations yielding saturable binding of Rab5 (75nM), and which stimulate endosome fusion. This resulted in increased endosome recruitment to microtubules. These observations suggest that Rab5 regulates the attachment of endosomes to microtubules. Experimental details are given in Example 5.

**Rab5 regulates early endosome motility on microtubules *in vitro.*** Next, the possibility that Rab5 may also control the motility of early endosomes on microtubules was investigated. For this, an assay was developed that reconstitutes this process *in vitro,* and performed analysis using time-lapse video microscopy. In this assay, a uniform lawn of taxol-stabilized microtubules was allowed to stick to the glass surfaces of a perfusion chamber. Microtubules were polymerized *in vitro* from purified, fluorescently labeled tubulin, and consisted of weakly fluorescent stretches of tubules interspersed with strongly labeled "seeds". Purified endosomes labeled with rhodamine-transferrin were mixed with cytosol, ATP, and an anti-fade mixture, and perfused into the chamber for analysis by video-microscopy. Significant numbers of fluorescently labeled endosomes were observed moving along microtubules in a cytosol- and ATP-dependent fashion. Interestingly, addition of 1µM RabGDI, resulted in an almost complete inhibition of endosome motility, indicating that Rab GTPases are required for this function. To determine whether Rab5 played a direct role in this process, a mutated form of Rab5, Rab5D136N, was utilized. Since this mutant binds xanthine rather than guanine nucleotides, it is possible to specifically modulate the nucleotide-bound state of this protein without interfering with other GTPases. Loading of endosomes with Rab5D136N in the presence of XDP inhibited endosome motility. In contrast, activation of Rab5D136N with XTPγS led to a ∼2.5-fold stimulation of motility events/min over cytosol. Neither XTPγS alone nor the late endosomal Rab protein, Rab7, could stimulate endosome motility in this assay. Therefore, Rab5 specifically regulates early endosome motility along microtubules.

Plus-ended motility of organelles on microtubules occurs through the action of motor proteins of the kinesin family, whereas minus-ended motility can occur through the action of either dynein motor proteins or specific members of the kinesin motor protein family. Since bi-directional motility of endosomes was observed, the inventors used function-blocking antibodies to gain insights into which of these motor protein families were responsible for the endosome movement *in vitro.* Addition of the dynein function blocking antibody, 70.1, did not alter the proportion of minus-ended versus plus-ended motility of endosomes. On the other hand, addition of MC44, an anti-kinesin antibody recognizing multiple forms of kinesin, inhibited motility in both directions. Experimental details are summarized in Example 6.

These preliminary results also indicate the involvement of a novel kinesin motor protein in the Rab5-dependent endosome motility. In other words, the kinesin itself is a suitable target. Alternatively, molecules that regulate the recruitment or activation of a motor may be the target. The identification of these protein(s) will provide potential novel targets for drug therapy against cell invasiveness.

### Class 6

### Representative effectors/regulators: Rab5 GAP (alternative designation: RN Tre) (Matoskova et al., 1996; Matoskova et al., 1996)

### Function: Rab5 GAP (GTPase Activating Protein) regulates the GTPase state of Rab5

### Assay: Rab5 GTPase activity

Since Rab5 regulates endocytosis effectors/regulators of this molecule also control endocytosis as well as all functions controlled by Rab5; i.e., endocytic trafficking, intracellular distribution of endosomes, motility of endosomes along microtubules, and cellular motility. The present inventors have discovered that RN Tre, a protein related to the *Tre* oncogene, is a Rab5 GAP, which discovery provides an interesting link between the signal transducion and membrane trafficking machinery. Most importantly, like Rabex-5 that regulates activation of Rab5, RN Tre regulates its inactivation and therefore represents an ideal target for drugs aimed at interfering (positively and negatively) with Rab5 function.

### Examples

### Example 1:

The following example describes the materials and methods used to measure the effect of a GTPase or mutant GTPase on the regulation of cell motility.

### Expression constructs

Myc-rhoDV26G (Murphy et al., 1996) was cloned into the HindIII-XbaI sites of expression vector pHSVPUC (Geller et al., 1993) to generate construct: pHSV-myc-rhoDV26G. Myc-rhoDV26G cDNA was cloned in frame into the EcoRI-BamHI sites of expression vector pEGFP-C2 (Clontech) to generate a green fluorescent protein (GFP) fusion: GFP-myc-RhodV26G.

### Transient transfection of rhoDV26G and human transferrin receptor

BHK-21 cells were trypsinized 24h prior to transfection and were seeded onto 11mm glass coverslips. Cells were infected with T7 RNA polymerase recombinant vaccinia virus (Bucci et al., 1992) and transfected with either T7-myc-rhoDV26G and T7-human transferrin receptor (T7-hTR) or T7-hTR alone (Murphy et al., 1996) using DOTAP (Boehringer Mannheim, Mannheim, Germany). The cell number and also the amount of DNA and lipid were constant. The final concentration of DNA per coverslip was 1µg.

Following transfection, the cells were incubated at 37°C, 5% CO₂, for 2h, cyclohexamide was then added for 1h. Cells were washed 8 times for 1min in preheated medium and rhodamine transferrin (50 µg/ml) was uptaken in a preheated humidified chamber at 37°C, 5% CO₂, for 20min. Coverslips were washed in preheated medium and then mounted into chambers for video microscopy (Bradke and Dotti, 1997) as described below. TLVM (time lapse video microscopy) was carried out immediately. Hydroxyurea was present at all times to prevent late viral gene expression.

### Transient transfection of T7-rhoDV26G and T7-gfp

To monitor lysosomal motility in the presence and absence of rhoDV26G we made use of a green fluorescent protein-expressing vector cloned into pGEM1 harboring the T7 promoter. Co-transfection of this plasmid with T7-myc-rhoDV26G allowed us to identify the transfected cells for video microscopy. Handling of the cells was as outlined above with the exception that they were incubated in the presence of 50 nM Lysotracker (Molecular Probes) for 15min at 37°C, 5% CO₂ to label the lysosomes, and TLVM was carried out as outlined below.

### BBCE cell motility

To address the effect of rhoDV26G on cell motility, BBCE cells were trypsinized and plated at a density of 1,500 cells per cm², 24h later nuclear microinjection was carried out with a RhoD V26G expression construct at 50µg/ml concentration, FITC dextran was coinjected to identify the injected cells. Approximately, 12h following injection the cells were stimulated with bFGF and subjected to TLVM.

### Time-Lapse Video Microscopy

The cells for TLVM were grown on coverslips and mounted into aluminum chambers as described in Bradke and Dotti (1997). Briefly, aluminum was cut into 7.5cm x 2.5cm x 2mm rectangles, the dimensions are similar to a standard glass slide and fit onto the microscope stage. In the center a circular hole was cut of 8mm diameter, and around it 4mm area was milled from both sides. A glass coverslip was inserted onto one side of the chamber, sealed with a lubricant, medium was then added to fill the chamber, cells grown on coverslips were then inverted onto the other side of the metal slide and sealed with lubricant.

### Image and statistical analysis of the data

A dedicated automatic program was developed to detect and track endosomes/lysosomes as they move. It runs on a SPARC station Ultral (SUN, Mountain View, CA) to which a Series 151/40 digital image processor (Imaging Technology, Bedford, MA) is connected. The detection of fluorescent spots corresponding to endosomes/lysosomes is performed automatically by a multiresolution algorithm based upon selectively filtering an undecimated wavelet decomposition of the image through the use of wavelet coefficient thresholding and correlation (Olivo, 1996)). At the end of this step, all endosomes/lysosomes in the sequence are characterized and their coordinates are determined and stored. A tracking algorithm is then used to establish valid trajectories. The algorithm uses a first-order Kalman filtering approach whereby at each frame and on the basis on the previous ones, predictions of the endosome/lysosome positions are established and compared with the computed ones (Nguyen Ngoc et al., 1997). The best matches are selected as trajectory points and tracks are finally analyzed to compute the data that was used for generating values in Tables 1 and 2. For each data set, a sequence of 30 images was analyzed. Speed values reported here are mean values of the speed of several endosomes/lysosomes and errors are standard deviations of these group estimulates.

### Example 2:

Drugs that have been found by employing the assay of the present invention and that enhance or inhibit Rab5 effector/regulator activity can be distinguished and classified on the basis of their mode of action. Accordingly, 5 classes of drugs will be introduced (and searched for) that exhibit a specific mode of action on the target molecules, i.e., on the effectors or regulators:
1. Drugs that inhibit Rab5 activation. Such drugs would in principle act on the whole Rab5 network and are therefore less specific than drugs of classes 3 and 4 because they act on a regulator (protein or molecule) that affects the nucleotide state of Rab5 and, consequently, the ability of Rab5 to signal to the totality of Rab5 effectors rather than on a specific effector (protein or molecule).
2. Drugs that enhance Rab5 inactivation. Such drugs would in principle act on the whole Rab5 network and are less specific than drugs of classes 3 and 4 for the reason given for class 1 above.
3. Drugs that disrupt specific Rab5-effector interactions, e.g., between Rab5 and p110 FYVE.
4. Drugs that inhibit a specific Rab5 effector activity, e.g., endosome fusion, endosome-microtubule interaction, endosome motility along microtubules.
5. Drugs that inhibit cell motility.

Class 3 and 4 drugs are the most specific and are therefore preferred according to the present invention. However, drugs of the other classes, in particular of classes 1 and 2, can nevertheless be considered as suitable anti-cancer and/or anti-infectious diseases pharmaceuticals.

### Assay for Activity 1

The assay measures Rabex-5 dependent nucleotide exchange of Rab5. The drug could perturb Rab5-Rabex-5 interaction or inhibit the catalytic activity of Rabex-5. The result expected is an inhibition of GDP/GTP exchange of Rab5. This assay is based on the assay published in (Horiuchi et al., 1997) and may well be adequately adapted for high throughput screening. It will be readily feasible, therefore, to screen thousands of compounds for their potential anti-cancer or anti-infectious disease activity/potential.

GDP/GTP exchange activity. Unless otherwise specified, the standard [³⁵S]GTPγS-binding assay was performed by the filter method (Sasaki et al., 1990) by incubating 200nM of recombinant Rab5 with 1µM of [³⁵S]GTPγS (20,000cpm/pmol) at 37°C for 10min with 5mM of MgCl₂ in the presence of 20nM recombinant Rabex-5.

Substances that are able to enhance or inhibit nucleotide exchange above or below the standard activity measured in the presence of Rabex-5, respectively, may be searched for.

### Assay for Activity 2

The assay measures the GTPase Activating Protein (GAP) RN-Tre-dependent stimulation of GTP hydrolysis by Rab5. The drug could stabilize the Rab5-GAP interaction or stimulate the catalytic activity of GAP. The result expected is an increase in the inactive-form of Rab5, i.e., an increased amount of GDP-bound Rab5. This assay is based on the following assay:

GAP assays with purified recombinant RN-Tre fusion proteins were performed by the filter method (Sasaki et al., 1990) using 2µM of substrate [γ-³²P]-GTP-loaded Rab5 in the presence of recombinant RN Tre GAP domain (100nM), at 30°C for 2min. GAP activity was expressed as the percentage of non-hydrolyzed [γ-³²P]-GTP which remained bound to the filters, relative to the radioactivity at time 0. Substances that are able to increase the GTP hydrolysis activity compared with the standard activity measured in the presence of RN Tre should be searched for. The assay may adequately be adapted for high throughput screening.

### Assay for Activity 3

Drugs may be screened for their ability to inhibit specific Rab5-Rab5 effector interactions. The assay consists in the binding of an effector molecule (either *in vitro* translated or as purified recombinant protein; native or modified, e.g., by biotinylation) to GST-Rab5:GTPgS (GTPgammaS) immobilized on a matrix. This assay is based on the procedure developed by Christoforidis et al. (1999a and b), but may be readily adapted for high throughput screening.

GST-Rab5 affinity chromatography is performed as described previously (Chistoforidis et al., 1999a). Beads with GST-Rab5 were incubated with the nucleotide exchange buffer (NE buffer) containing 20mM Hepes, 100mM NaCl, 10mM EDTA, 5mM MgCl₂, 1mM DTT, 1mM GTPγS, pH7.5, for 90min at room temperature under rotation. Afterwards, NE buffer was removed and the nucleotide was stabilized with NS buffer containing 20mM Hepes, 100mM NaCI, 5mM MgCl₂, 1mM DTT, 1mM GTPγS, pH7.5, for 20min at room temperature under rotation. *In vitro* transcription-translation of ³⁵S-methionine labeled proteins is performed according to Manufacturer's instructions (Promega). Recombinant proteins or transcribed-translated proteins (50µl of standard reactions) are incubated for 2h at 4°C with 20µl glutathione sepharose beads coupled with GST-Rab5:GTPgS, and 150µl buffer containing 20mM Hepes, 100mM NaCI, 5mM MgCl₂, 1mM DTT, and 1mM GTPgS, respectively, such that the final volume is 220µl. The incubation is performed both in the presence and in the absence of the candidate drug. When the drug is dissolved in an organic solvent the buffer in the control reaction will be supplemented with the solvent in the same concentration and amount added in the reaction in the presence of the drug. Usually, however, the buffer/solvent dissolving the drug is the same buffer used for the assay, i.e., the buffer containing 20mM Hepes, 100mM NaCl, 5mM MgCl₂, 1mM DTT, and 1mM GTPgS. Beads are subsequently washed 4x with buffer containing 20mM Hepes, 100mM NaCl, 5mM MgCl₂, 1mM DTT, 10µM GTPgS, lx with the same buffer but now containing 250mM NaCl, and 1x with 20mM Hepes, 250mM NaCI, 1mM DTT. Elution of bound proteins is performed as described before (Chistoforidis et al., 1999a; Christoforidis and Zerial, 1999) and eluted proteins are loaded on SDS-PAGE gel followed by immunoblotting for recombinant proteins or autoradiography for *in vitro* translated proteins.

The assay measures the activity of drugs that are capable of disrupting the association of a specific or any Rab5 effector with Rab5 in a concentration-dependent manner under the same experimental conditions that allow the interaction. For example, a drug x will disrupt the interaction of effector X with Rab5:GTPgS in a concentration-dependent manner. This drug will then be used as inhibitor of effector X in the biological process X.

A variation of the assay is to use magnetic beads in an assay of the ELISA-type (similar to the *in vitro* endosome fusion assay described below). GST-Rab5 was biotinylated using chemical crosslinking (Pierce) and nucleotide exchange was performed. Beads with GST-Rab5 were incubated with NE buffer containing 20mM Hepes, 100mM NaCl, 10mM EDTA, 5mM MgCl₂, 1mM DTT, 1mM GTPgS, pH7.5, for 90min at room temperature under rotation. Afterwards, the NE buffer was removed and the nucleotide was stabilized with NS buffer containing 20mM Hepes, 100mM NaCl, 5mM MgCl₂, 1mM DTT, 1mM GTPgS, pH7.5, for 20min at room temperature under rotation. Beads (1µl) were subsequently incubated with 1µg recombinant purified Rab5 effector coupled to a ruthenium trisbipyridine chelate (IGEN) in 20µl NS buffer in the presence or absence of chemical drug candidates. Beads were then washed three times with 500µl NS buffer and binding of the effector to Rab5 is measured with an Origen Analyzer (IGEN) according to the manufacturer procedure. The background signal (binding to Rab5:GDP, i.e., without nucleotide exchange) is deducted. Drugs that affect the interaction between Rab5 and the Rab5 effector decrease the signal in comparison with the signal obtained when the reaction is performed in the absence of the drug.

The assay measures the activity of drugs that are capable of disrupting the association of a specific or any Rab5 effector with Rab5 in a concentration-dependent manner under the same experimental conditions that allow the interaction. For example, a drug x will disrupt the interaction of effector X with Rab5:GTPgS in a concentration-dependent manner. This drug will then be used as inhibitor of effector X in the biological process X.

### Assays for Activity 4

The present inventors have developed three main assays to measure Rab5 and Rab5 effector activity.

### (i) In vitro fusion assay (Horiuchi et al., 1997)

This assay is intended to screen for drugs that inhibit endocytosis, clathrin coated vesicle (CCV) fusion with early endosomes, early endosome homotypic fusion and endosome trafficking in tumor cells or enhance endocytic membrane fusion in bacterial parasites infected cells.

Two distinct enriched populations of early endosomes labelled with either biotinylated transferrin or a sheep α-human transferrin antibody are prepared from sHeLa (suspension HeLa) cells by sucrose sedimentation (Gorvel et al., 1991). The basal fusion reaction consists of the two enriched endosome populations incubated for 45minutes at 37°C in the presence of 3mg/ml of HeLa cytosol, unlabelled transferrin and an ATP regeneration system (17.3mM creatine phosphate, 87µg/ml creatine kinase, and 2.2mM ATP) and in the presence or absence of the candidate drug. The fusion is quantified by incubation of the fusion mix with wash buffer (50mM Tris pH7.5, 100mM NaCI, 1g/l BSA and 2% (w/v) Triton X-100) and streptavidin-coated magnetic beads (Dynal). After two washes in wash buffer the samples are incubated with a rabbit α-sheep secondary antibody coupled to a ruthenium trisbipyridine chelate (IGEN) and measured with an Origen Analyzer (IGEN). The background signal (5-10% of the fusion signal) is deducted and the data expressed as the percentage of the basal fusion reaction. The CCV-early endosome fusion assay is performed in the same way with biotinylated tranferrin-labelled CCV and α-tranferrin antibody-labelled early endosomes.

Substances that are able to inhibit CCV-endosome or early endosome fusion below the standard fusion activity in the presence of cytosol and an ATP-regenerating system may be searched for. To control that the inhibition is targeted to a Rab5 effector (by inhibiting the Rab5-Rab5 effector interaction or the Rab5 effector activity), the inhibitory effect of the drug should be rescued by increasing concentrations of that particular Rab5 effector. For this a titration curve of the drug in the presence of increasing concentrations of the Rab5 effector will be necessary.

The following two assays are intended to screen for drugs that inhibit endosome movement and cell motility in tumor cells or enhance endocytic membrane fusion in infected cells. The assays score the overall activity, but as soon as the effector molecules specifically involved in these processes are identified, it will provide the additional possibility of using Assay for activity 3.

### (ii) Microtubule spin-down assay

75 to 100µg purified early endosomes are incubated at room temperature for 20min with 100µg HeLa cytosol protein in a reaction brought to a final volume of 50µl by addition of BRB-80 (80mM K-Pipes, 1mM MgCl₂, 1mM EGTA, pH6.8). 10µl of taxol-stabilized microtubules (100µg tubulin equivalent) are added, and this mix is incubated for 10min at room temperature, in the presence or absence of the candidate drug, and then overlayed on 600µl of a 35% (w/v) sucrose cushion. After sedimentation at 100,000 x g for 20min at 22°C in a TLA 100.4 rotor, the upper layer was removed, the cushion was washed with 100µl BRB-80 and then also removed. The remaining pellet was resuspended in 30µl 2x SDS-PAGE buffer plus 30µl dH₂O, and analyzed by SDS-PAGE and immunoblotting.

Substances that are able to inhibit the association of early endosomes with microtubules below the standard fusion activity in the presence of cytosol and an ATP-regenerating system may be searched for. To control that the inhibition is targeted to a Rab5 effector (by inhibiting the Rab5-Rab5 effector interaction or the Rab5 effector activity), the inhibitory effect of the drug may be rescued by increasing concentrations of that particular Rab5 effector. For this a titration curve of the drug in the presence of increasing concentrations of the Rab5 effector is necessary.

### (iii) In vitro endosome motility assay

Early endosomes and cytosol are isolated as previously described (Horiuchi et al., 1997), except that HeLa spinner culture cells were allowed to internalize rhodamine-labeled transferrin for 10min at 37°C in order to label early endosomes. Oregon-green labeled tubulin is polymerized *in vitro,* and the resulting microtubules isolated, stabilized with taxol, and perfused into a microscope slide/glass coverslip chamber as previously described (Marlowe and al., 1998). The chamber is then washed with BRB-80 plus 10µM taxol to stabilize microtubules and remove non-polymerized tubulin. A mixture of HeLa cytosol (2mg/ml), MgATP (200µM), fluorescently-labeled endosomes (3mg/ml), purified bovine hemoglobin (3mg/ml; Sigma), and an anti-fade system (Howard and Hyman, 1993), in the presence or absence of the candidate drug, is perfused into the chamber, and images of fluorescently-labeled microtubules (FITC filter set) or endosomes (Rhodamine filter set) are collected at 2sec-intervals using a time-lapse fluorescence videomicroscope. All image acquisition, processing, and analysis of movies is performed using the NIH Image v1.60 software package. Endosome movements are defined as linear, vectorial motions that occur on fluorescent microtubules over four or more consecutive images. These are discriminated from tethered Brownian motions, which generally display non-linear shaking, or flip randomly back and forth from image to image. Movements are counted from at least three and in most cases many more, individual movies, averaged and population significant differences calculated using the Excel spreadsheet program (Microsoft, Inc.). Substances that are able to inhibit the motility of early endosomes with microtubules below the standard fusion activity in the presence of cytosol and an ATP-regenerating system can be searched for. To control that the inhibition is targeted to a Rab5 effector (by inhibiting the Rab5-Rab5 effector interaction or the Rab5 effector activity), the inhibitory effect of the drug may be rescued by increasing concentrations of that particular Rab5 effector. For this a titration curve of the drug in the presence of increasing concentration of the Rab5 effector is necessary.

### Assay for activity 5

This assay describes the measurement of cell motility in vitro. The assay may be standardized for high throughput screening.

To address the effect of drugs on cell motility, BBCE cells or other tumor cell lines are trypsinized and plated at a density of 1,500 cells per cm². 24h later the cells are stimulated with bFGF or other growth factors and subjected to Time-Lapse Video Microscopy (TLVM). The cells for TLVM are grown on coverslips and mounted into aluminum chambers as described in (Bradke and Dotti, 1997)). Briefly, aluminum is cut into 7.5cm x 2.5cm x 2mm rectangles, the dimensions are similar to a standard glass slide and fit onto the microscope stage. In the center a circular hole is cut of 8 mm diameter, and around it 4mm area is milled from both sides. A glass coverslip is inserted onto one side of the chamber, sealed with a lubricant, medium is then added to fill the chamber, cells grown on coverslips are then inverted onto the other side of the metal slide and sealed with lubricant. TLVM is carried out using a standard video microscopy set up. A dedicated automatic program was developed to detect and track fluorescently labeled cells as they move.

### Example 3:

A novel Procedure for the Purification of Rab5 effectors.

1201 of bacterial DH5α cells were grown to express GST-Rab5 for the large scale isolation of Rab5 effectors. The cells were induced, harvested, and broken according to manufacturer instructions (Pharmacia). Lysis buffer (2500ml) consisted of a PBS solution containing 100µM GDP, 5mM MgCl₂, 5mM 2-mercaptoethanol, 5µg/ml DNAase, 5µg/ml RNAase, and a cocktail of protease inhibitors. High speed supernatant of the cell lysate was incubated with 20ml glutathione sepharose beads (Pharmacia) at 4°C for 2h under rotation. Subsequently, beads were loaded on a column and washed with lysis buffer without DNAase, RNAase, and protease inhibitors. This procedure resulted in 1g of GST-Rab5 bound to the column. The molecule is recovered in the GDP-bound inactive form and nucleotide exchange is necessary to convert it into the GTP-bound form to allow effector binding.

The present inventors have designed an alternative procedure that overcomes the low efficiency normally affecting this step. The modification consists in the use of aluminum fluoride (AlF₃) which when bound to Ras:GDP and Mg²⁺ has been shown to mimick the transition state for GTP-hydrolysis of the GTPase complexed to GAP (Scheffzek et al., 1997). The inventors have obtained evidence, however, that the method can also be applied for the complex between Rab5:GDP:Mg²⁺:AlF₃ and its effectors. In other words, it is possible to obtain a complex consisting of Rab5, GDP, Mg²⁺, AlF₃, and Rab5 effectors. This procedure has for example allowed the inventors to purify at large scale EEA1 and Rabankyrin-5, suggesting that this method can be applied for the identification of novel effectors and regulators for small GTPases.

### Example 4:

### Construction and expression of EGFP-Rab5

pEGFP-Rab5 was constructed by PCR amplification of the human Rab5a cDNA from pGEM-myc-Rab5 using the primers CCCAAGCTTATGGCTAGTCGAGGCGCAACA and AACTGCAGTTAGTTACTACAACACTGATT followed by cloning of the HindIII-PstI fragment from the PCR product into a pEGFP-C3 expression vector (Clontech, Inc.). A431 cells were grown to ∼80% confluency in 10cm petri dishes and transfected with 20-30µg of plasmid DNA using a calcium phosphate based protocol. Stably transfected clonal lines were isolated after incubation in selective (G418; 0.5µg/ml) growth medium for 7-10d and checked for GFP fluorescence on endosomal structures. Despite the presence of the selectable marker, variable levels of expression of EGFP-Rab5 in these cells were observed.

### Time-lapse fluorescence videomicroscopy of EGFP-Rab5 in vivo

Cells were grown on glass coverslips and were transferred to custom-built aluminum microscope slide chambers (EMBL workshop, Heidelberg) just prior to observation. Unless otherwise stated, cells expressing average levels of EGFP-Rab5 were selected and analyzed on a Zeiss Axioskop microscope using 100X/1.40 plan-Apochromat lens with a temperature-controlled objective sleeve attached (EMBL workshop, Heidelberg). Time-lapse imaging was performed, collecting images at 2sec-intervals using a computer-controlled shutter (Uniblitz, Inc.) with illumination by a 100W mercury arc lamp attenuated with two heat reflection filters and a KG-1 heat absorbance filter (Zeiss). GFP-fluorescence was visualized with Hi-Q FITC, or GFP filter sets (Chroma Technologies, Inc.), and images were aquired using a COHU 4913 CCIR video camera with on-chip integration controlled by the NIH-Image vl.60 software package.

### Example 5:

### Microtubule spin-down assay

75 to 100µg of purified early endosomes were incubated at room temperature for 20min with 100µg HeLa cytosol protein in a reaction brought to a final volume of 50µl by addition of BRB-80 (80mM K-Pipes, 1mM MgCl₂, 1mM EGTA, pH6.8). 10µl of taxol-stabilized microtubules (100µg tubulin equivalent) was added and this mix was incubated for 10min at room temperature, and then overlayed on 600µl of a 35% (w/v) sucrose cushion. After sedimentation at 100,000 x g for 20min at 22°C in a TLA 100.4 rotor, the upper layer was removed, the cushion was washed with 100µl BRB-80 and then also removed. The remaining pellet was resuspended in 30µl 2x SDS-PAGE buffer plus 30µl dH₂0, and analyzed by SDS-PAGE and immunoblotting.

### Example 6:

### In vitro endosome motility assays

Early endosomes, and cytosol were isolated as previously described, except HeLa spinner culture cells were allowed to internalize rhodamine-labeled transferrin for 10minat 37°C in order to label early endosomes. Oregon-green labeled tubulin was polymerized *in vitro,* and the resulting microtubules isolated, stabilized with taxol, and perfused into a microscope slide/glass coverslip chamber as previously described. Alternatively, purified centrosomes were allowed to stick to the glass surfaces of the chamber. Microtubule asters were then polymerized *in situ* by 30min incubation of the chamber at 37°C with fluorescently-labeled tubulin (4mg/ml) and 1mM GTP in BRB-80. The chamber was then washed with BRB-80 plus 10µM taxol to stabilize microtubules and remove non-polymerized tubulin. A mixture of HeLa cytosol (2mg/ml), MgATP (200µM), fluorescently-labeled endosomes (3mg/ml), purified bovine hemoglobin (3mg/ml; Sigma), and an anti-fade system was perfused into the chamber, and images of fluorescently-labeled microtubules (FITC filter set) or endosomes (Rhodamine filter set) were collected at 2sec-intervals using the time-lapse fluorescence videomicroscope setup described above.

### Analysis and quantification of videos

All image acquisition, processing, and analysis of movies was performed using the NIH Image v1.60 software package. Endosome movements were defined as linear, vectorial motions that occurred on fluorescent microtubules over four or more consecutive images. These were discriminated from tethered Brownian motions, which generally displayed non-linear shaking, or flipped randomly back and forth from image to image. Movements were counted from at least three and in most cases many more, individual movies, averaged and population significant differences calculated using the Excel spreadsheet program (Microsoft, Inc.).

### References

Alvarez-Dominguez, C., Barbieri, A. M., Beron, W., Wandinger-Ness, A., and Stahl, P. D. (1996). Phagocytosed live Listeria monocytogenes influences Rab5-regulated in vitro phagosome-endosome fusion. J. Biol. Chem. *271*, 13834-13843.

Alvarez-Dominguez, C., and Stahl, P. D. (1999). Increased expression of Rab5a correlates directly with accelerated maturation of Listeria monocytogenes phagosomes. J Biol Chem *274,* 11459-62.

Alvarez-Dominguez, C., and Stahl, P. D. (1998). Interferon-gamma selectively induces Rab5a synthesis and processing in mononuclear cells. J Biol Chem *273*, 33901-4.

Amano, M., Mukai, H., Ono, Y., Chihara, K., Matsui, T., Hamajima, Y., Okawa, K., Iwamatsu, A., and Kaibuchi, K. (1996). Identification of a putative target for Rho as the serine-threonine kinase protein kinase N. Science *271,* 648-650.

Bar-Sagi D, F. J. (1986). Induction of membrane ruffling and fluid-phase pinocytosis in quiescent fibroblasts by ras proteins. Science *233,* 1061-1068.

Bradke, F. and Dotti, C. (1997). Videomicroscopy of living microinjected hippocampal neurons in culture., A. Cid-Arregui and A. Garcia-Carranca, eds. (Heidelberg: Springer-Verlag), pp. 81-95.

Bretscher, M. S. (1992). Cells use their transferrin receptors for locomotion. EMBO J. *11,* 383-389.

Bretscher, M. S., and Aguado-Velasco, C. (1998). Membrane traffic during cell locomotion. Curr. Opin. Cell. Biol. *10*, 537-541.

Bucci, C., Parton, R. G., Mather, I. H., Stunnenberg, H., Simons, K., Hoflack, B., and Zerial, M. (1992). The small GTPase rab5 functions as a regulatory factor in the early endocytic pathway. Cell *70,* 715-728.

Cantley, L. C., Auger, K. R., Carpenter, C., Duckworth, B., Graziani, A., Kapeller, R., and Soltoff, S. (1991). Oncogenes and signal transduction. Cell *64*, 281-302.

Chistoforidis, S., McBride, H. M., Burgoyne, R., D., and Zerial, M. (1999a). The Rab5 effector EEA1 is a core component of endosome docking. Nature *397*, 621-625.

Christoforidis, S., Miaczynska, M., Ashman, K., Wilm, M., Zhao, L., Yip, S.-C., Waterfield, M. D., Backer, J. M., and Zerial, M. (1999b). Phosphoinositide-3-Kinases are Rab5 effectors. Nature Cell Biol. *4*, 249-252.

Christoforidis, S., and Zerial, M. (1999). An affinity chromatography approach leading to the purification and identification of novel Rab effectors. Methods in press.

Deretic V, F. R. (1999). Mycobacterium tuberculosis phagosome. Mol. Microbiol. *31*, 1603-1609.

Deretic V, V. L., Fratti RA, Deretic D (1997). Mycobacterial phagosome maturation, rab proteins, and intracellular trafficking. Electrophoresis *18*, 2542-2547.

Field, J., Vojtek, A., Ballester, R., Bolger, G., Colicelli, J., Ferguson, K., Gerst, J., Kataoka, T., Michaeli, T., Powers, S., Riggs, M., Rodgers, L., Wieland, I., Wheland, B., and Wigler, M. (1990). Cloning and Characterization of CAP, the S. cerevisiae Gene Encoding the 70 kd Adenylyl Cyclase-Associated Protein. Cell *61*, 319-327.

Geller, A. I., During, M. J., Haycock, J. W., Freese, A., and Neve, R. (1993). Long-term increases in neurotransmitter release from neuronal cells expressing a constitutively active adenylate cyclase from a herpes simplex virus type 1 vector. Proc Natl Acad Sci USA *90*, 7603-7.

Gorvel, J.-P., Chavrier, P., Zerial, M., and Gruenberg, J. (1991). Rab5 controls early endosome fusion in vitro. Cell *64*, 915-925.

Gournier, H., Stenmark, H., Rybin, V., Lippe, R., and Zerial, M. (1998). Two distinct effectors of the small GTPase Rab5 cooperate in endocytic membrane fusion. Embo J *17*, 1930-40.

Habets GG, S. E., Zuydgeest D, van der Kammen RA, Stam JC, Berns A, Collard JG (1994). Identification of an invasion-inducing gene, Tiam-1, that encodes a protein with homology to GDP-GTP exchangers for Rho-like proteins. Cell *77*, 537-549.

Hall, A. (1992). Ras-related GTPases and the cytoskeleton. Molecular Biology of the Cell *3*, 475-479.

Hopkins, C. R., Gibson, A., Shipman, M., Strickland, D. K. a., and Trowbridge, I. S. (1994). In migrating fibroblasts, recycling receptors are concentrated in narrow tubules in the pericentriolar area, and then routed to the plasma membrane of the leading lamella. J. Cell Biol. *125*, 1265-1274.

Horiuchi, H., Lippé, R., McBride, H. M., Rubino, M., Woodman, P., Stenmark, H., Rybin, V., Wilm, M., Ashman, K., Mann, M., and Zerial, M. (1997). A novel Rab5 GDP/GTP exchange factor complexed to Rabaptin-5 links nucleotide exchange to effector recruitment and function. Cell *90*, 1149-1159.

Howard, J., and Hyman, A. A. (1993). Preparation of marked microtubules for the assay of the polarity of microtubule-based motors by fluorescence microscopy. Methods Cell Biol. *39*, 105-113.

Ito, K., Ishii, N., Miyashita, A., Tominaga, K., Kuriyama, H., Maruyama, H., Shirai, M., Naito, M., Arakawa, M., and Kuwano, R. (1999). Molecular cloning of a novel 130-kDa cytoplasmic protein, Ankhzn, containing Ankyrin repeats hooked to a zinc finger motif. Biochem Biophys Res Commun *257*, 206-13.

Kramer, H., and Phistry, M. (1996). Mutations in the Drosophila hook gene inhibit endocytosis of the boss transmembrane ligand into multivesicular bodies. J Cell Biol *133*, 1205-15.

Machesky, L. M., and Hall, A. (1996). Rho: a connection between membrane receptor signalling and the cytoskeleton. Trends Cell Biol. *6*, 304-310.

Malonne, H., Langer, I., Kiss, R., and Atassi, G. (1999). Mechanisms of tumor angiogenesis and therapeutic implications: angiogenesis inhibitors. Clin Exp Metastasis *17*, 1-14.

Marlowe, K. J., and al., e. (1998). Changes in kinesin distribution and phosphorylation occur during regulated secretion in pancreatic acinar cells. Eur. J. Cell Biol. *75*, 140-152.

Matoskova, B., Wong, W. T., Nomura, N., Robbins, K. C., and Di Fiore, P. P. (1996). RN-tre specifically binds to the SH3 domain of eps8 with high affinity and confers growth advantage to NIH3T3 upon carboxy-terminal truncation. Oncogene *12*, 2679-88.

Matoskova, B., Wong, W. T., Seki, N., Nagase, T., Nomura, N., Robbins, K. C., and Di Fiore, P. P. (1996). RN-tre identifies a family of tre-related proteins displaying a novel potential protein binding domain. Oncogene *12*, 2563-71.

Michiels F, H. G., Stam JC, van der Kammen RA, Collard JG (1995). A role for Rac in Tiam1-induced membrane ruffling and invasion. Nature *375,* 338-340.

Murphy, C., Grummt, M., Saffrich, R., Gournier, H., Rybin, V., Rubino, M., Auvinen, P., Parton, R. G., and Zerial, M. (1996). Endosome dynamics regulated by a novel rho protein. Nature *384*, 427-432.

Nguyen Ngoc, S., Briquet-Laugier, F., Boulin, C., and Olivo, J.-C. (1997). Proceedings of IEEE International Conference on Image Processing ICIP'97,. In IEEE International Conference on Image Processing ICIP'97, (Santa Barbara, CA, pp. 484-487.

Novick, P., and Zerial, M. (1997). The diversity of Rab proteins in vesicle transport. Curr. Op. Cell Biol. *9*, 496-504.

Olivo, J.-C. (1996). IEEE International Conference on Image Processing ICIP'96, (Lausanne, pp. 311-314.

Peterson, M. R., Burd, C. G., and Emr, S. D. (1999). Vaclp coordinates Rab and phosphatidylinositol 3-kinase signaling in Vps45p-dependent vesicle docking/fusion at the endosome. Curr Biol *9*, 159-62.

Rothman, J. E. (1994). Mechanisms of intracellular protein transport. Nature *372,* 55-63.

Sasaki, T., Kikuchi, A., Araki, S., Hata, Y., Isomura, M., Kuroda, S., and Takai, Y. (1990). Purification and characterization from bovine brain cytosol of a protein that inhibits the dissociation of GDP from and the subsequent binding of GTP to smg p25A, a ras-like GTP-binding protein. J. Biol. Chem. *265,* 2333-2337.

Scheffzek, K., Ahmadian, M. R., Kabsch, W., Wiesmuller, L., Lautwein, A., Schmitz, F., and Wittinghofer, A. (1997). The Ras-RasGAP complex: structural basis for GTPase activation and its loss in oncogenic Ras mutants. Science *277,* 333-8.

Simonsen, A., Lippé, R., Christoforidis, S., Gaullier, J.-M., Brech, A., Callaghan, J., Toh, B.-H., Murphy, C., Zerial, M., and Stenmark, H. (1998). EEA1 links phosphatidylinositol 3-kinase function to Rab5 regulation of endosome fusion. Nature *394*, 494-498.

Sorkin, A., and Waters, C. M. (1993). Endocytosis of growth factor receptors. Bioessays *15,* 375-382.

Stenmark, H., Parton, R. G., Steele-Mortimer, O., Lütcke, A., Gruenberg, J., and Zerial, M. (1994). Inhibition of rab5 GTPase activity stimulates membrane fusion in endocytosis. EMBO J. *13*, 1287-1296.

Stenmark, H., Vitale, G., Ullrich , O., and Zerial, M. (1995). Rabaptin-5 is a direct effector of the small GTPase Rab5 in endocytic membrane fusion. Cell *83*, 423-432.

TerBush, D. R., Maurice, T., Roth, D., and Novick, P. (1996). The Exocyst is a multiprotein complex required for exocytosis in Saccharomyces cerevisiae. Embo J *15,* 6483-94.

Tsukazaki, T., Chiang, T. A., Davison, A. F., Attisano, L., and Wrana, J. L. (1998). SARA, a FYVE domain protein that recruits Smad2 to the TGFbeta receptor. Cell *95,* 779-91.

Veithen A, A. M., Van Der Smissen P, Cupers P, Courtoy PJ (1998). Regulation of macropinocytosis in v-Src-transformed fibroblasts: cyclic AMP selectively promotes regurgitation of macropinosomes. J. Cell Sci. *111,* 2329-2335.

Vieira, A. V., Lamaze, C., and Schmid, S. L. (1996). Control of EGF receptor signaling by clathrin-mediated endocytosis. Science *274*, 2086-9.

Zelicof, A., Protopopov, V., David, D., Lin, X.-Y., Lustgarten, V., and Gerst, J. E. (1996). Two separate functions are encoded by the carboxyl-terminal domains of the yeast Cyclase-associated protein and its mammalian homologs. J. Biol. Chem. *271*, 18243-18252.

## Claims

1. Use of an effector of a GTPase as a target in an *in vitro* or *in vivo* assay to detect substances useful as pharmaceutical agents for the prophylaxis and/or treatment of cancers, inflammations, allergies, skin repair diseases such as psoriasis and/or infectious diseases.

2. Use of claim 1, wherein the GTPase is of the Rab or Rho family.

3. Use of claim 2, wherein the GTPase is Rab4, Rab5, Rab 11, Rab 17, or RhoD.

4. Use of any of the preceding claims, wherein the infectious disease is tuberculosis, pseudotuberculosis, cholera, malaria, gastroenteritis, enteric fever, typhus, those diseases caused by pathogens (bacteria or organisms) such as Mycobacterium, Staphylococcus, Toxoplasma, Trypanosoma, Listeria, Salmonella, Legionella, Leishmania, Coxiella, Shigella, Yersinia, Neisseria, Vibrio, Bartonella, or any other infectious disease caused by an infectious agent that infects cells by the endocytic route and resides intracellularly in phagosomes escaping the cellular killing mechanisms.

5. Use of any of claims 1 to 3, wherein the cancer is a benign tumor, a malignant tumor, a carcinoma, a sarcoma, a melanoma, a leukemia, a glioma, or a neuroblastoma, in particular a lung carcinoma, an osteosarcoma, a lymphoma, a soft tissue sarcoma, a breast carcinoma, a bile cancer, a cervix carcinoma, a cancer of the (small) intestine, of the kidneys, of the cavity of the mouth, a penis carcinoma, an ovary cancer, a stomach cancer, a cancer of the tongue, a brain cancer, a bladder carcinoma, a prostate carcinoma, a liver carcinoma, a carcinoma of the pancreas, and every tumor that invades other tissues and organs distinct from its site of origin.

6. Use of any of the preceding claims, wherein the assay is carried out in the presence of one or more GTPase effector/regulator molecule(s) which is/are either native and biochemically purified from a vertebrate, or recombinant and biochemically purified from bacterial cultures, from yeast cultures, or from other cultured eukaryotic cells, in either case labelled by a covalent modification or radioactivity suitable for use in the assay.

7. Use of claim 6, wherein the assay is carried out in the simultaneous presence of at least one type of GTPase and/or endosomal membrane fractions fluorescently labeled or labeled by any other modification that allows its detection, and/or cytosolic extracts, and/or an ATP-regenerating system and/or a number of chemicals to be tested for their suitability as an anti-cancer or anti-infectious diseases drug.

8. Use of claim 7, wherein the at least one GTPase is a GTPase selected from Rab 4, Rab 5, Rab 11, Rab 17, or RhoD.

9. Use of any of the preceding claims, wherein the substance useful as pharmaceutical agent is a molecule/substance that carries one or more of the following functional groups: a halide atom bound to an alkyl, alkenyl, alkinyl or aryl residue, an alcohol group, an ether group, a carbonyl function (aldehyde or ketone), a carboxylic acid group, a carboxylic anhydride group, a carbamoyl group, a haloformyl group, a cyano group, an ester group including a lactone group, a benzyl, phenyl, tolyl, tosyl, sulfonyl group, an amino group (primary, secondary, tertiary), an isocyanate, a cyanate, a thioisocyanate, a thiocyanate, a carbamate, an azide, a diazo group, and a quinone group; or is an organometallic compound, a sterol moiety(ies)-containing molecule, an inorganic acid or complex such as a metallocene, a cytokine, a hormone, or an oligopeptide comprising up to 20, preferably 8, 10, or 12, amino acid residues.

10. A kit useful for carrying out the assay of any of the preceding claims, the kit comprising in a suitable means at least one GTPase effector/regulator molecule which is either native and biochemically purified from a vertebrate, or recombinant and biochemically purified from bacterial cultures, from yeast cultures, or from other cultured eukaryotic cells, in either case labelled by a covalent modification or by radioactivity suitable for the use in the assay.

11. The kit of claim 10, wherein the kit further comprises at least one type of GTPase and/or endosomal membrane fractions fluorescently labeled or labeled by any other modification that allows its detection, and/or cytosolic extracts, and/or an ATP-regenerating system and/or a number of chemicals to be tested for their suitability as an anti-cancer or anti-infectious diseases drug.

12. The kit of claim 11, wherein the at least one GTPase is/are one or more of the GTPases Rab 4, Rab 5, Rab 11, Rab 17, or RhoD.
